(19) Europäisches Patentamt
European Patent Office
Office européen des brevets

(11) Numéro de publication: **0 269 514 B1**

# (12) FASCICULE DE BREVET EUROPEEN

(45) Date de publication de fascicule du brevet: **26.08.92**

(51) Int. Cl.[5]: **C07C 309/63**, C07C 61/40, C07C 62/02, C07C 69/743, C07C 69/757, C07D 317/24, C07F 9/09, C07F 9/6574, C07F 9/6578, C07C 255/00

(21) Numéro de dépôt: **87402605.7**

(22) Date de dépôt: **19.11.87**

Demande divisionnaire 91105956.6 déposée le 19/11/87.

## (54) Nouveaux dérivés de l'acide 2,2-diméthyl cyclopropane carboxylique portant en 3 une chaîne halogénée saturée, leur procédé et les intermédiaires de préparation, leur application comme pesticides et les compositions les renfermant.

(30) Priorité: **20.11.86 FR 8616155**

(43) Date de publication de la demande: **01.06.88 Bulletin 88/22**

(45) Mention de la délivrance du brevet: **26.08.92 Bulletin 92/35**

(84) Etats contractants désignés: **AT BE CH DE ES FR GB GR IT LI LU NL**

(56) Documents cités:
**EP-A- 0 187 674**
**EP-A- 0 217 342**

**CHEMICAL ABSTRACTS, vol. 86, no. 19, 9 mai 1977, page 513, résumé no. 139480a, Columbus, Ohio, USA & JP-A-76 122 041**

(73) Titulaire: **ROUSSEL-UCLAF**
**35, Boulevard des Invalides**
**F-75007 Paris(FR)**

(72) Inventeur: **Cadiergue, Joseph**
**6, rue Jules Princet**
**F-93600 Aulnay-sous-Bois(FR)**
Inventeur: **Demoute, Jean Pierre**
**249 bis, rue de Rosny**
**F-93100 Montreuil-Sous-Bois(FR)**
Inventeur: **Tessier, Jean**
**30, rue Jean Moulin**
**F-94300 Vincennes(FR)**

(74) Mandataire: **Tonnellier, Marie-José et al**
**Département des Brevets ROUSSEL UCLAF**
**B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 269 514 B1

**TETRAHEDRON LETTERS, vol. 27, no. 19, 1986, pages 2139-2142, Pergamon Journals Ltd., GB; M. FUJITA et al, "Practical and stereocontrolled syntheses of both (1R*, 3S*)- and (1R*, 3R*)-3-(2-chloro-3,3,3-trifluoro-1-propenyl)-2,-2-dimethylcyclopropanecarboxylates"**

**TETRAHEDRON LETTERS, vol. 27, no. 19, 1986, pages 2135-2138, Pergamon Journals Ltd., GB; M. FUJITA et al, "Efficient carbon-carbon bond formation with thermally stable 1,1-dihalo-2,2,2-trifluoroethylzinc reagent"**

## Description

La présente invention concerne de nouveaux dérivés de l'acide 2,2-diméthyl cyclopropanecarboxylique portant en 3 une chaîne halogénée saturée, leur procédé de préparation et leur application comme pesticides.

On connaissait (Chemical Abstracts (1977) 86 p. 513 n° 39840 et Tetrahedron Letters (1986) 27 N° 19 p. 2139-2142) et EP 217342 des dérivés de l'acide cyclopropanecarboxylique portant en 3 une chaîne

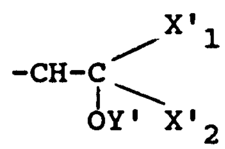

respectivement précurseurs d'esters d'acide 2,2-diméthyl-3-(2,2-dihalovinyl) cyclopropanecarboxylique.

L'invention a pour objet les composés de formule (I) :

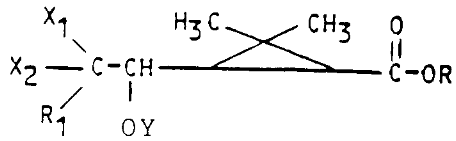 (I)

dans laquelle $X_1$ et $X_2$ identiques ou différents l'un de l'autre représentent un atome d'halogène, $R_1$ représente un atome d'halogène, un radical alkyle renfermant jusqu'à 8 atomes de carbone, un radical aryle renfermant jusqu'à 14 atomes de carbone éventuellement substitué, un radical -C≡N, un radical

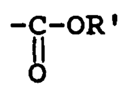

dans laquelle R' représente un radical alkyle renfermant jusqu'à 8 atomes de carbone, Y représente un radical choisi dans le groupe constitué par :

- les radicaux $SO_2alc_1$, $alc_1$ représentant un radical alcoyle

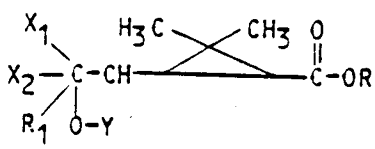 (I)

dans laquelle $X_1$ et $X_2$ identiques ou différents l'un de l'autre représentent un atome d'halogène, $R_1$ représente un atome d'halogène, un radical alkyle renfermant jusqu'à 8 atomes de carbone, un radical aryle renfermant jusqu'à 14 atomes de carbone éventuellement substitué, un radical perfluoroalkyle renfermant jusqu'à 8 atomes de carbone, un radical -C≡N, un radical

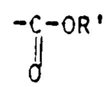

dans laquelle R' représente un radical alkyle renfermant jusau'à 8 atomes de carbone, Y représente un radical choisi dans le groupe constitué par :

- les radicaux $SO_2alc_1$, $alc_1$ représentant un radical alcoyle linéaire ou ramifié, saturé ou insaturé éventuellement substitué par un ou plusieurs groupements fonctionnels ;
- les radicaux $SO_2A_r$, $A_r$ représentant un radical aryle renfermant jusqu'à 14 atomes de carbone, éventuellement substitué par un ou plusieurs groupements fonctionnels ;

- les radicaux

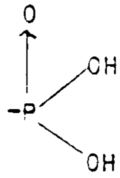 ou 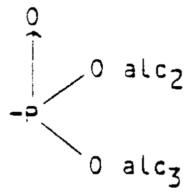

$alc_2$ et $alc_3$ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou $alc_2$ et $alc_3$ pouvant former avec le radical

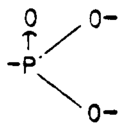 ,

un cycle

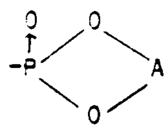

A représentant une chaîne carbonée linéaire ou ramifiée, saturée ou insaturée éventuellement substituée par un ou plusieurs groupements fonctionnels et renfermant jusqu'à 6 atomes de carbone ;
- les radicaux

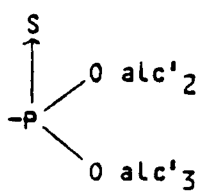 ,

$alc'_2$ et $alc'_3$ représentant les mêmes valeurs que $alc_2$ et $alc_3$ ;
- le radical

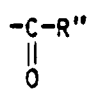 ,

R" représentant un radical aryle et R représente
- soit un atome d'hydrogène,
- soit un radical alkyle, linéaire ou ramifié, éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

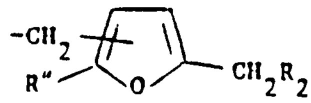

dans lequel le substituant R" représente un atome d'hydrogène ou un radical méthyle et le substituant $R_2$ un aryle monocyclique ou un groupement -C≡CH et notamment un groupement 5-benzyl 3-furyl méthyle,

- soit un groupement :

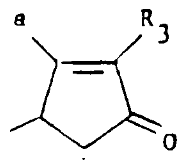

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C{\equiv}CH$,

- soit un groupement :

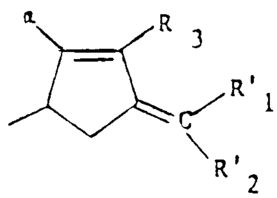

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

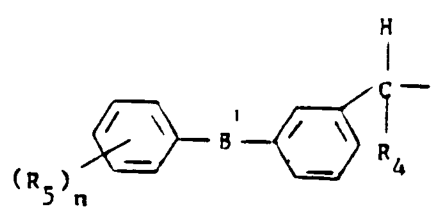

dans lequel B' représente un atome d'oxygène ou de soufre, un groupement

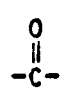

ou $-CH_2$ ou un groupement sulfoxyde ou un groupement sulfone et $R_4$ représente un atome d'hydrogène, un radical -C≡N, un radical méthyle, un radical $-CONH_2$, un radical $-CSNH_2$ ou un radical -C≡CH, $R_5$ représente un atome d'halogène ou un radical méthyle et n représente un nombre égal à 0,1 ou 2, et notamment le groupement 3-phénoxy benzyle, alpha-cyano 3-phénoxy

benzyle, alpha-éthynyl 3-phénoxy benzyle, 3-benzoyl benzyle, 1-(3-phénoxy phényl) éthyl ou alpha-thioamido 3-phénoxy benzyle,

- soit un groupement :

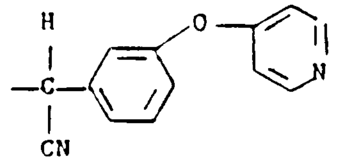

- soit un groupement :

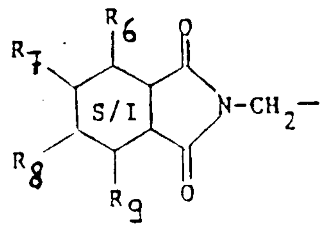

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

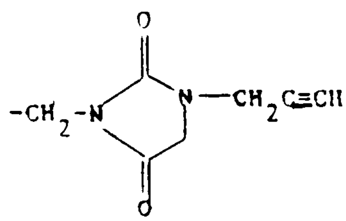

- soit un groupement :

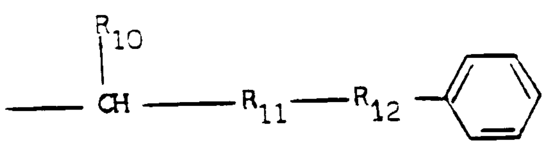

dans lequel $R_{10}$ représente un atome d'hydrogène ou un radical CN, $R_{12}$ représente un radical $-CH_2$ ou un atome d'oxygène, $R_{11}$ représente un radical thiazolyle ou thiadiazolyle dont la liaison avec

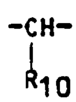

peut se trouver à l'une quelconque des positions disponibles, $R_{12}$ étant lié à $R_{11}$ par l'atome de carbone compris entre l'atome de soufre et un atome d'azote,

- soit un groupement :

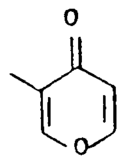

- soit un groupement :

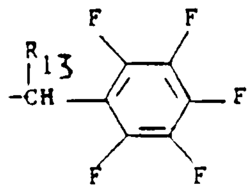

dans lequel $R_{13}$ représente un atome d'hydrogène ou un radical CN,

- soit un groupement :

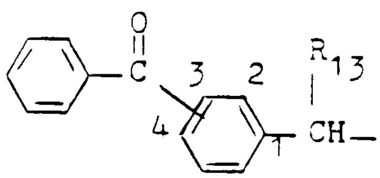

dans lequel $R_{13}$ est défini comme ci-dessus, et le radical benzoyle est en position 3 ou 4,

- soit un groupement :

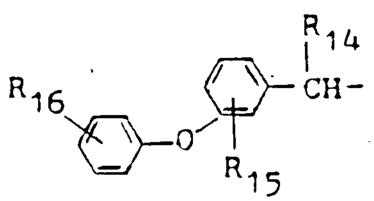

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupement :

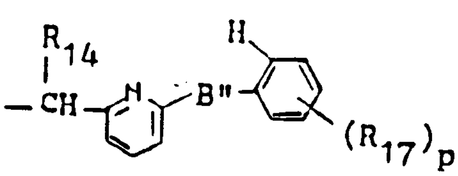

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et 8" représente un atome d'oxygène ou un atome de soufre,

- soit un groupement :

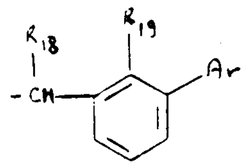

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,

- soit un groupement :

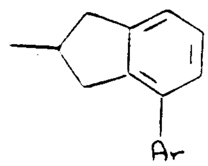

ou

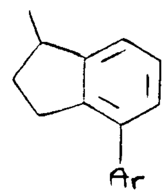

- soit un groupement :

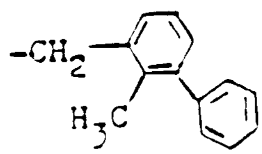

- soit un groupement :

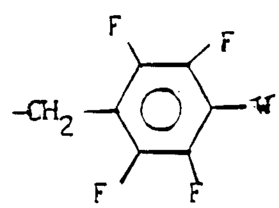

avec W = H, $CH_3$, $OCH_3$

à la condition que $R_1$ ne représente pas un radical $CF_3$ si R = N alkyle.

Les composés de formule I présentent plusieurs centres d'asymétrie, les carbones en 1 et 3 du cyclopropane et les carbones en 1' et 2' de la chaîne latérale

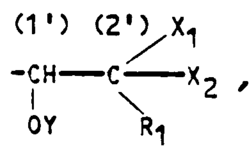

ils peuvent présenter également plusieurs centres d'asymétrie dans la partie R. L'invention a pour objet les différents stéréoisomères possibles ainsi que les mélanges de ces stéréoisomères.

$X_1$ et $X_2$ identiques ou différents représentent de préférence un atome de chlore, de brome ou d'iode.

Lorsque $R_1$ représente un atome d'halogène, il s'agit de préférence d'un atome de brome, de chlore ou de fluor.

Lorsque $R_1$ représente un radical alkyle, il s'agit de préférence du radical méthyle, éthyle, n-propyle,

isopropyle, n-butyle, isobutyle ou terbutyle.

Lorsque $R_1$ représente un radical aryle, il s'agit de préférence du radical phényle.

Lorsque $R_1$ représente un radical aryle substitué, il s'agit de préférence du radical phényle substitué par un atome d'halogène, par exemple un atome de chlore.

Comme combinaisons préférées de $X_1$, X et $R_1$, on peut citer les combinaisons suivantes :

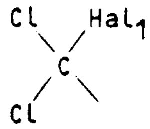

avec $Hal_1$ = Br, I

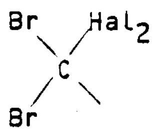

avec $Hal_2$ = Br, I

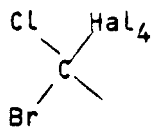

avec $Hal_4$ = Br, I

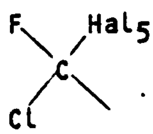

avec $Hal_5$ = Br, I

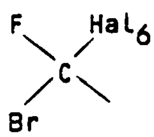

avec $Hal_6$ = Br,

$Alc_1$, $alc_2$, $alc_3$, $alc'_2$ et $alc'_3$ représentent de préférence un radical méthyle, éthyle, n-propyle, isopropyle, n-butyle, isobutyle, t-butyle.

Ar représente de préférence un radical phényle.

Lorsque $alc_1$, $alc_2$, $alc_3$, $alc'_2$, $alc'_3$ et Ar sont substitués, ils le sont de préférence par un ou plusieurs des substituants suivants : les atomes d'halogène, les radicaux $CF_3$, hydroxyle, carboxyle, amino, ammonium, les radicaux alkyle et alkoxy renfermant jusqu'à 4 atomes de carbone, par exemple les radicaux méthyle et méthoxy.

A représente de préférence une chaîne carbonée saturée renfermant de 1 à 4 atomes de carbone, éventuellement substituée par un ou plusieurs radicaux choisis dans le groupe suivant ; les radicaux alkyles renfermant de 1 à 5 atomes de carbone, les atomes d'halogène, les radicaux $CF_3$ ou les radicaux hydroxyles.

Lorsque R représente un radical alkyle, il s'agit de préférence du radical méthyle, éthyle, n-propyle, isopropyle, butyle, isobutyle, terbutyle.

Les valeurs préférées des alcools utilisés dans la synthèse des esters pyréthrinoïdes seront indiquées ci-après.

L'invention a plus particulièrement pour objet les composés dans lesquels $X_1$ et $X_2$ représentent un atome de chlore, de brome ou d'iode, par exemple les composés dans lesquels $X_1$ et $X_2$ représentent un atome de brome ainsi que les composés dans lesquels $R_1$ représente un atome de brome, de chlore ou du

fluor.

Parmi les composés préférés de l'invention, on peut citer les composés dans lesquels Y représente un radical $SO_2alc_1$, $alc_1$ représentant un radical alkyle ou alkényle renfermant jusqu'à 8 atomes de carbone par exemple un radical $SO_2CH_3$, les composés dans lesquels Y représente un radical $SO_2A_{r'}$. $A_r$ conservant la même signification que précédemment, ainsi que les composés de formule I dans lesquels R représente :

- soit un atome d'hydrogène,
- soit un radical alkyle, linéaire ou ramifié, éventuellement subtitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

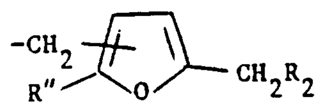

dans lequel le substituant R" représente un atome d'hydrogène ou un radical méthyle et le substituant $R_2$ un aryle monocyclique ou un groupement -C≡CH et notamment un groupement 5-benzyl 3-furyl méthyle,

- soit un groupement :

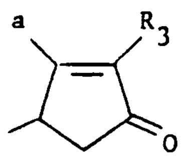

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- soit un groupement :

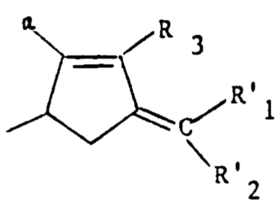

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

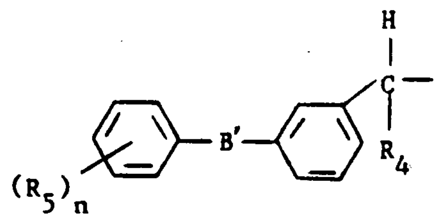

dans lequel B' représente un atome d'oxygène ou de soufre, un groupement

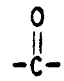

ou $-CH_2$-ou un groupement sulfoxyde ou un groupement sulfone et $R_4$ représente un atome d'hydrogène, un radical -C≡N, un radical méthyle, un radical $-CONH_2$, un radical $-CSNH_2$ ou un radical -C≡CH, $R_5$ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,

- soit un groupement :

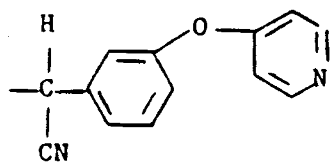

- soit un groupement :

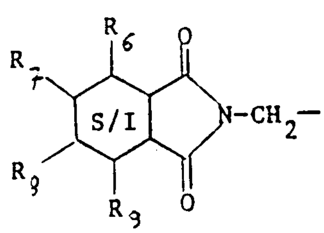

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

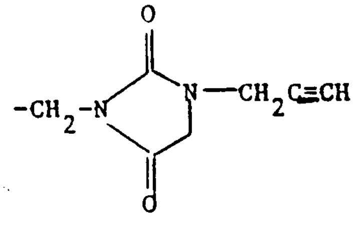

- soit un groupement :

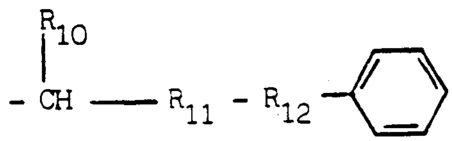

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec

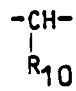

peut se trouver à l'une quelconque des positions disponibles, $R_{12}$ étant lié à $R_{11}$ par l'atome de carbone compris entre l'atome de soufre et un atome d'azote,

- soit un groupement :

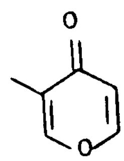

- soit un groupement :

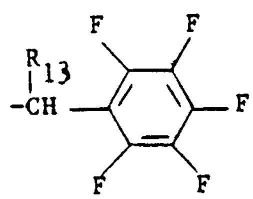

dans lequel $R_{13}$ représente un atome d'hydrogène ou un radical CN,

- soit un groupement :

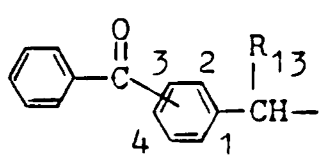

dans lequel $R_{13}$ est défini comme ci-dessus, et le radical benzoyle est en position 3 ou 4,

- soit un groupement :

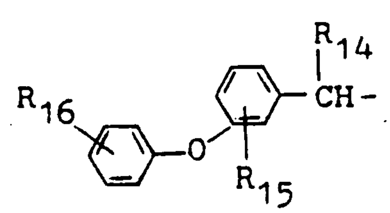

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupement :

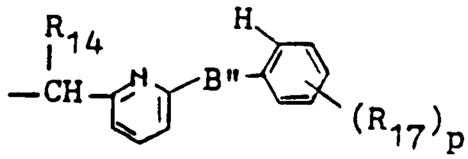

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylènedioxy, chloro, fluoro ou bromo, p représente un nombre égal à 0,1 ou 2 et B" représente un atome d'oxygène ou un atome de soufre,

- soit un groupement :

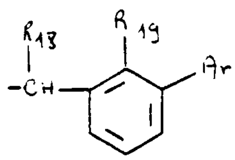

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,

- soit un groupement :

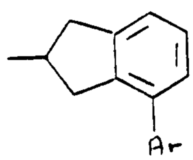

ou

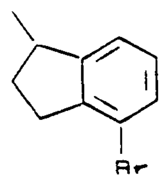

Parmi les composés préférés de l'invention, on peut citer tout spécialement les composés de formule I dans lesquels R représente un radical :

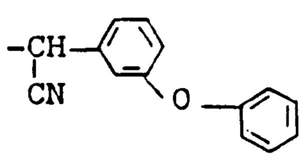

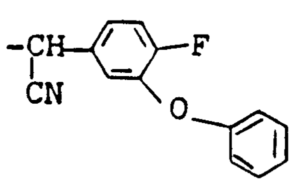

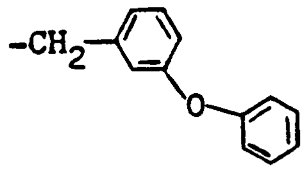

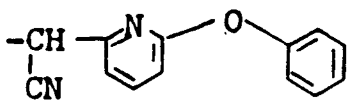

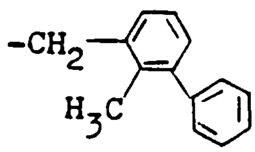

ou 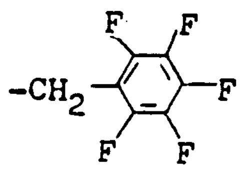 ou 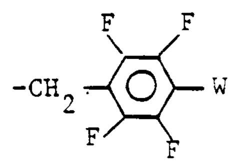

avec W=H, $CH_3$, $OCH_3$

ou 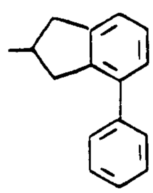

L'invention a plus spécialement pour objet les composés de formule suivants :

- le 1R-[1-alpha-(S) 3-alpha-(R)] 2,2-diméthyl 3-[1-(méthylsulfonyloxy) 2,2,2,-tribromoéthyl] cyclopropane carboxylate de cyano (3-phénoxyphényl) méthyle,
- le 1R-[1-alpha-(S) 3-alpha-(R)] 2,2-diméthyl 3-[2,2,2-tribromo 1-méthyl sulfonyloxy éthyl] cyclopropane carboxylate de cyano [(3-phénoxy 4-fluoro)phényl] éthyle,
- le 1R-[1-alpha, 3-alpha (RS*,RS*)] 2,2-diméthyl 3-[2-trifluorométhyl 2-bromo 2-chloro 1-méthyl sulfonyloxyéthyl] cyclopropane carboxylate de cyano (3-phénoxyphényl) méthyle.

L'invention a également pour objet un procédé de préparation, caractérisé en ce que l'on soumet un composé de formule II :

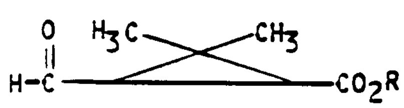 (II)

dans laquelle R conserve la même signification que précédemment à l'action d'un composé de formule V :

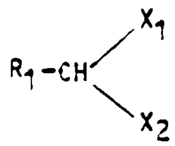

(V)

dans laquelle $X_1$ et $X_2$ conservent la même signification que précédemment et $R_1$ conserve la même signification que précédemment en présence d'une base pour obtenir le composé de formule IV :

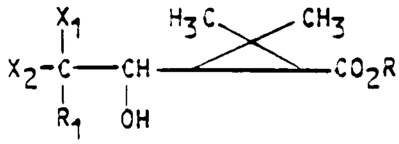

(IV)

que l'on soumet à l'action d'un agent de sulfonylation ou de phosphonylation ou de thiophosphorylation ou d'acylation pour obtenir le composé ce formule I correspondant :

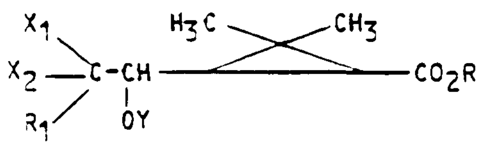

que l'on transforme éventuellement en acide correspondant que l'on soumet éventuellement à un agent d'estérification pour obtenir un nouveau composé de formule I.

Dans un mode de réalisation préféré du procédé de l'invention :

- la base utilisée lors de la réaction du composé de formule II avec le composé de formule V est de préférence choisie dans le groupe constitué par les alcoolates alcalins, les hydrures alcalins et les hydroxydes alcalins. Comme bases tout particulièrement préférées, on peut citer la potasse, le méthylate ou le terbutylate de potassium ;
- L'agent de sulfonylation répond à la formule (A)

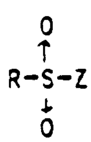

avec Z =
( halogène
( COalc (alc 1 à 8 C)
( $OSO_2$alc (alc 1 à 8 C)
( imidazolyle),

- L'agent de phosphonylation répond à la formule (B)

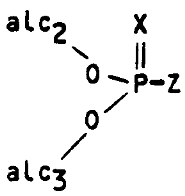

avec Z=
( halogène
( $OSO_2R$
( imidazolyle

X représent eun atome d'oxygène ou de soufre et $alc_2$ et $alc_3$ conservent la même signification que dans la définition des produits de formule (I) ;
- l'agent d'acylation répond à la formule

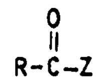

avec Z = 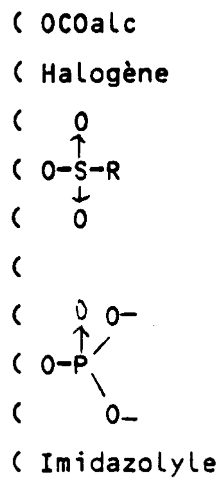

- l'agent de formule I peut être préparé en faisant réagir un ester de formule I avec un agent d'hydrolyse acide, par exemple l'acide paratoluènesulfonique, l'acide sulfurique, l'acide acétique ;
- L'agent d'estérification est un alcool, l'estérification étant réalisée selon les méthodes classiques.

Les produits de formule IV obtenus lors de la mise en oeuvre du procédé sont des produits nouveaux à l'exception des produits pour lesquels $X_1$, $X_2$ et $R_1$ représentent chacun un atome d'halogène.

Les composés de formule (I) présentent d'intéressantes propriétés qui permettent leur utilisation dans la lutte contre les parasites ; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites des animaux à sang chaud.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les composés de L'invention présentent des propriétés insecticides tout à fait remarquables, en particulier un très bon pouvoir de knock down.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Parmi les compositions notamment insecticides préférées de l'invention, on peut citer tout spécialement les compositions renfermant les composés décrits dans les exemples et notamment :

- le 1R-[1-alpha-(S) 3-alpha-(R)] 2,2-diméthyl 3-[1-(méthylsulfonyloxy) 2,2,2,-tribromoéthyl] cyclopropane carboxylate de cyano (3-phénoxyphényl) méthyle,

- le 1R-[1-alpha-(S) 3-alpha-(R)] 2,2-diméthyl 3-[2,2,2 tribromo 1-méthyl sulfonyloxy éthyl] cyclopropane carboxylate de cyano [(3-phénoxy 4-fluoro)phényl] éthyle,
- le 1R-[-alpha, 3-alpha (RS*,RS*)] 2,2-diméthyl 3-[2-trifluorométhyl 2-bromo 2-chloro 1-méthyl sulfonyloxyéthyl] cyclopropane carboxylate de cyano (3-phénoxyphényl) méthyle.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appats ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active, est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin), amidon et poudre de coque de noix de coco. La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95% en poids.

L'invention a également pour objet, les compositions acaricides renfermant comme principe actif au moins des produits de formule (I) définie ci-dessus.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrage foliaires contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

L'orsqu'il s'agit de lutter contre les parasites des animaux, les compositions peuvent être utilisées sous forme de spray, de bains, ou enore selon la méthode "pour-on".

Lorsqu'on utilise la méthode pour-on, on utilise de préférence des solutions renfermant de 0,5 g à 4 g de principe actif pour 100 cm3 de solution.

Lorsqu' il s'agit de lutter contre les acariens parasites des animaux, on incorpore très souvent les produits de l'invention dans des compositions alimentaires en association avec un mélange nutritif adapté à

l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des anti-oxydants.

L'invention a donc également pour objet les compositions destinées à l'alimentation animale, renfermant comme principe actif au moins l'un des produits de formule (I) telle que définie précédemment.

On peut indiquer également que les produits de L'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcool-alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido-méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2,-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présentent notamment l'intérêt, soit de permettre de combattre, par la polyvalence de leur action, une gamme de parasites, plus étendue, soit de manifester, dans certains cas, un effet de synergie.

Pour exalter l'activité biologique des produits de l'invention, on peut les additionner à des synergistes classiques utilisés en pareil cas tel le 1-(2,5,8-trioxadodécyl) 2-propyl 4,5-méthylènedioxy benzène (ou butoxyde de pipéronyle) ou la N-(2-éthylheptyl) bicyclo [2,2,-1] 5-heptène-2,3-dicarboximide, ou le pipéronyl-bis-2-(2'-n-butoxyéthoxy) éthylacétal (ou tropital).

L'invention a pour objet les compositions pesticides définies précédemment, caractérisées en ce qu'elles renferment en outre un synergiste des pyréthrinoïdes.

L'invention a également pour objet l'application des composés de formule I caractérisée en ce que l'on soumet un composé de formule I à l'action d'un agent de réduction pour obtenir le composé de formule III correspondant :

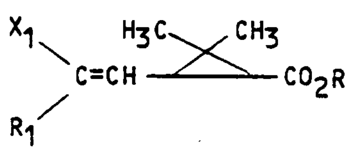

(III)

dans laquelle $X_1$, $R_1$ et R conservent leur signification précédente.

Dans un mode de réalisation préférée, l'agent de réduction utilisé est l'hydrogène en présence d'un catalyseur d'hydrogénation comme le palladium. On peut également effectuer cette réduction avec du zinc en présence d'un acide. On peut aussi utiliser le couple zinc-cuivre au sein d'un alcool ou toute autre méthode permettant d'éliminer simultanément un atome d'halogène et le groupe OY.

Comme application, particulièrement intéressante, on peut citer l'application caractérisée en ce que le composé de formule I répond à la formule I" :

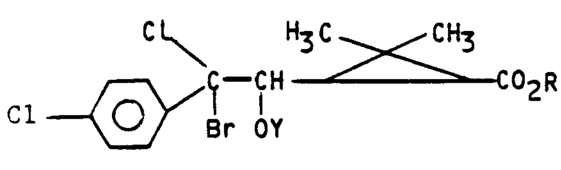

(I")

dans laquelle Y et R conservent la même signification que précédemment et le composé III obtenu à la formule III" :

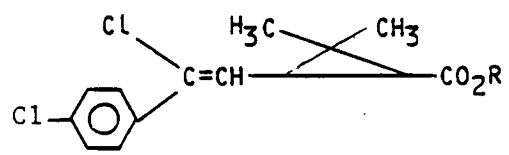

(III")

Les exemples suivants illustrent l'invention sans toutefois la limiter.

**Exemple 1 : 1R-[1-alpha-(S) 3-alpha-(R)] 2,2-diméthyl 3-(1-hydroxy 2,2,2-tribromoéthyl)cyclopropane carboxylate de cyano (3-phénoxy phényl) méthyle.**

**Stade A** : Acide 1R-[1-alpha-3-alpha-(R)] 2,2-diméthyl 3-[1-triméthyl silyloxy) 2,2,2-tribromoéthyl] cyclopropane carboxylique.

On introduit à -60¤C en 30 minutes dans une solution renfermant 30,25g d'acide 1R-[1-alpha 3-alpha-(R) 2,2-diméthyl 3-[1-hydroxy 2,2,2-tribromoéthyl] cyclopropane carboxylique décrit dans le brevet français 2396006 et 300cm3 de tétrahydrofuranne, 18,5g de terbutylate de potassium, 40cm3 de tétrahydrofuran et 80cm3 d'alcool terbutylique. On agite pendant 10 minutes à -60¤C et introduit goutte à goutte 80cm3 de chlorure de triméthyl silyle et 60cm3 de tétrahydrofuranne. On agite 10 minutes à -60¤C et laisse remonter la température à -20¤C. On agite ensuite une demi-heure à -20¤C. On verse le milieu réactionnel sur une solution saturée de bicarbonate de sodium. On extrait au chloroforme, sèche et amène à sec sous pression réduite. On ajoute 100cm3 d'eau à 1 % d'acide acétique. On agite 15 minutes, essore le produit obtenu, le dissout dans le chloroforme, sèche et amène à sec s pression réduite. On obtient ainsi 16g de produit fondant à 147¤C.

**Stade B :** 1R-[1-alpha-(S) 3-alpha-(R)] 2,2-diméthyl 3-[1-triméthyl silyloxy) 2,2,2-tribromoéthyl] cyclopropane, carboxylate de cyano (3-phénoxyphényl) méthyle.

On introduit une solution de 14g de dicyclohexyl carbodiimide et 140 cm3 de chlorure de méthylène dans une solution renfermant 32g de produit préparé au stade A, 300 cm3 de chlorure de méthylène et 15g d'alcool (S) alpha-cyano 3-phénoxylbenzylique. On agite pendant 3 heures à 20-25¤C. On essore et amène à sec sous pression réduite. On purifie par chromatographie en éluant par le mélange hexane-éther isopropylique 8-2. On obtient 34g de produit recherché fondant à 98¤C.

**Stade C :** 1R-[1-alpha-(S) 3-alpha-(R)] 2,2-diméthyl 3-(1-hydroxy 2,2,2-tribromoéthyl) cyclopropane carboxylate de cyano (3-phénoxyphényl) méthyle.

On ajoute 3 cm3 d'acide chlorhydrique 2N dans une solution renfermant 8g de produit obtenu au stade B de l'exemple 1 et 150 cm3 de méthanol. On agite 30 minutes à 20-25¤C et chasse le méthanol. On reprend par du chlorure de méthylène, sèche et amène à sec sous pression réduite. On obtient un produit que l'on chromatographie sur milieu en éluant par le mélange hexane-acétate d'éthyle 7-3. On obtient ainsi 6,5g de produit recherché.

spectre RMN $CDCl_3$ ppm

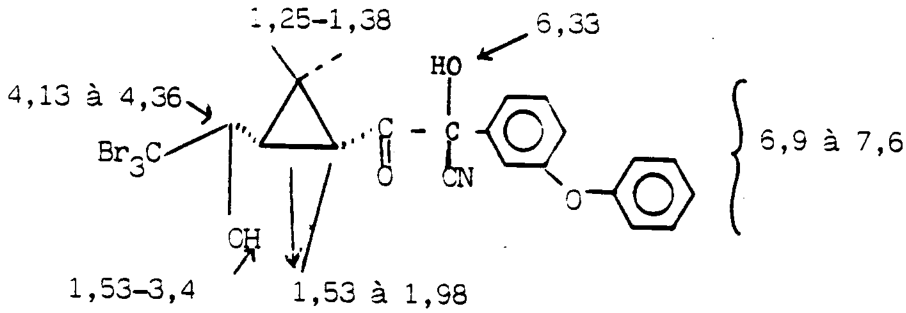


**Exemple 2 : 1R-[1-alpha-(S) 3-alpha-(R)] 2,2-diméthyl 3-[1-(méthylsulfonyloxy) 2,2,2-tribromoéthyl] cyclopropane carboxylate de cyano (3-phénoxy phényl méthyle.**

On introduit à 0¤ ± 5¤C 0,91 cm3 de triéthylamine dans un mélange de 3g de produit préparé à l'exemple 1, 30cm3 de tétrahydrofuran 0,5cm3 de chlorure de mésyle. On agite 2 heures à 0 ± 5¤C. On ajoute à nouveau 0,5cm3 de chlorure de mésyle et 0,91 cm3 de triéthylamine. On essore le précipité formé, lave la phase organique à l'eau, sèche et amène à sec sous pression réduite. On chromatographie sur silice un éluant par le mélange hexaneacétate d'éthyle 7-3. On obtient 2,6g de produit recherché.

Spectre de RMN $CDCl_3$ ppm

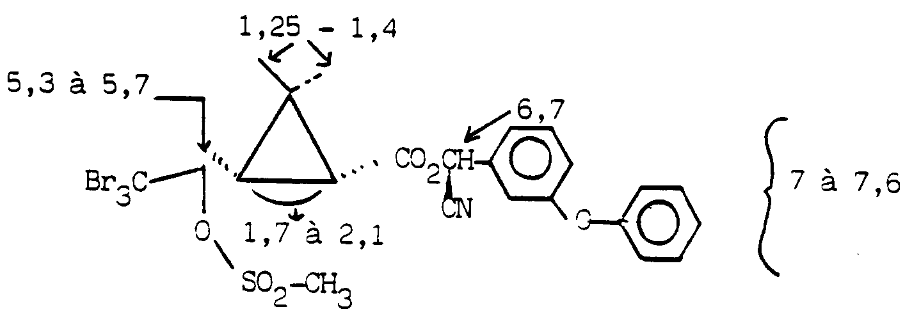


**Example 3 : 1R-[1-alpha-(S*) 3-alpha-(R*)] 2,2-diméthyl 3-(2,2,2-tribromo 1-éthyl sulfonyl oxyéthyl) cyclopropane carboxylate de cyano 1-(3-phénoxy phényl) méthyle.**

En opérant comme à l'exemple précédent au départ du produit préparé à l'exemple 1 et de chlorure d'éthane sulfonyle, on obtient le produit recherché fondant à 133¤C.
$[alpha]_D$ = +43¤ c = 1% $CHCl_3$

**Exemple 4 : 1R-[1-alpha-(S) 3-alpha-(R)] 3-(2,2,2-tribromo 1-méthyl éthyl sulfonyloxyéthyl) 2,2-diméthyl cyclopropane carboxylate de cyano 1-(3-phénoxyphényl) méthyle.**

On introduit à la température de 0 ± 5¤C, 0,35 cm3 de triéthylamine dans une solution renferment 1,5g du produit obtenu à l'exemple 1, 15cm3 de tétrahydrofuran et 0,32g de chlorure d'isopropylsulfinyle. On agite à 0±5¤C pendant 30 minutes. On verse le mélange réactionnel dans une solution aqueuse d'acide chlorhydrique à pH 5, extrait au chlorure de méthylène, sèche, filtre et évapore à sec. On introduit 1,5g d'acide paranitroperbenzoïque. On agite 30 minutes à 20-25¤C et amène à sec. On obtient 3,2g de produit recherché brut que l'on reprend par un mélange d'hexane-acide acétique 8-2 et chromatographie sur silice en éluant par le mélange hexane-acide acétique 8-2. On obtient 1,43g de produit recherché.
$[alpha]_D$ = +42¤ ± 1,5 c = 0,7%

**Exemple 5 : 1R-[1-alpha-(S) 3-alpha-(R)] 3-[2,2,2-tribromo 1-(2-méthyl 2-propène 1-sulfonyloxyéthyl] 2,2-diméthyl cyclopropane carboxylate de cyano (3-phénoxy phényl) méthyle.**

A partir du produit obtenu à l'exemple 1 et du chlorure de (1-sulfonyl oxy 2-méthyl) 2-propényle, en opérant comme à l'exemple 2, on obtient le produit recherché. F = 107¤C.

**Exemple 6 : 1R-[1-alpha-(R,S) 3-alpha-(R*)] 2,2-diméthyl 3-[2,2,2-tribromo 1-méthylsulfonyloxyéthyl] cyclopropane carboxylate d'alpha-(R,S) cyano 1-(6-phénoxy 2-pyridyl) méthyle.**

**Stade A :** 1R-[1-alpha-(R,S) 3-alpha-(R*)] 2,2-diméthyl 3-(2,2,2,-tribromo 1-triméthylsilyloxyéthyl) cyclopropane carboxylate d'alpha(R,S) cyano 1-(6-phénoxy 2-pyridyl) méthyle.

En opérant comme au stade B de l'exemple 1, à partir de l'acide 1R-[1-alpha, 3-alpha (R)] 2,2-diméthyl 3-(triméthyl silyloxy 2,2,2-tribromo éthyl cyclopropane carboxylique obtenu au stade A de l'exemple 1 et de l'alcool alpha-(R,S) cyano 1-(6-phénoxy 2-pyridyl) méthylique, on obtient le produit recherché fondant à 160¤C.

**Stade B :** 1R-[1-alpha-(R,S) 3-alpha-(R*)] 2,2-diméthyl 3-[2,2,2-tribromo 1-méthylsulfonyloxyéthyl] cyclopropane carboxylate d'alpha-(R,S) cyano 1-(6-phénoxy 2-pyridyl) méthyle.

En opérant comme à l'exemple 1, stade C, à partir du produit obtenu au stade A du présent exemple, on obtient le produit recherché que l'on utilise tel quel au stade suivant.

**Stade C :** 1R-[1-alpha-(R,S) 3-alpha-(R*)] 2,2-diméthyl 3-[2,2,2-tribromo 1-méthylsulfonyloxyéthyl] cyclopropane carboxylate d'alpha-(R,S) cyano 1-(6-phénoxy 2-pyridyl) méthyle.

En opérant comme à l'exemple 2 à partir du produit obtenu au stade B et du chlorure de mésyle, on obtient le produit recherché.
$[alpha]_D$ = +6¤ ± 1,5¤ c = 0,3% $CHCl_3$.

**Exemple 7 : 1R-[1-alpha, 3-bêta-(R)] 2,2-diméthyl 3-(2,2,2-tribromo 1-méthylsulfonyloxyéthyl cyclopropane carboxylate de (3-phénoxyphényl) méthyle.**

**Stade A :** 1R-[1-alpha, 3-alpha-(R)] 2,2-diméthyl 3-(2,2,2-tribromo 1-triméthylsilyloxyéthyl) cyclopropane carboxylate de (3-phénoxy phényl) méthyle.

En opérant comme à l'exemple précédent, à partir du produit obtenu au stade A de l'exemple 1 et de l'alcool métaphénoxy benzylique, on obtient le produit recherché.

**Stade B :** 1R-[1-alpha, 3-alpha-(R*)] 2,2-diméthyl 3-(2,2,2-tribromo 1-hydroxyéthyl) cyclopropane carboxylate de (3-phénoxyphényl) méthyle.

En opérant comme à l'exemple précédent stade B, à partir du produit obtenu au stade A, on obtient le produit recherché que l'on utilise tel quel dans le stade suivant.

**Stade C :** 1R-[1-alpha, 3-alpha (R)] 2,2-diméthyl 3-(2,2,2-tribromo 1-méthyl sulfonyloxy éthyl) cyclopropane carboxylate de (3-phénoxyphényl) méthyle.

En opérant comme à l'exemple précédent, stade C, à partir du produit obtenu au stade B du présent exemple, on obtient le produit recherché fondant à 72¤C.

**Exemple 8 : 1R-[1-alpha-(S) 3-alpha-(R)] 2,2-diméthyl 3-[2,2,2-tribromo 1-méthylsulfonyloxyéthyl] cyclopropane carboxylate de (S) cyano [(3-phénoxy 4-fluoro) phényl] méthyle.**

**Stade A :** 1R-[1-alpha-(S) 3-alpha-(R)] 3-[2,2,2-tribromo 1-triméthyl silyloxyéthyl] 2,2-diméthyl cyclopropane carboxylate de cyano [(3-phénoxy 4-fluoro) phényl] méthyle.

En opérant comme à l'exemple 7, à partir de l'acide 1R-[1-alpha, 3-alpha (R)] 2,2-diméthyl 3-[1-triméthyl silyloxy 2,2,2-tribromoéthyl] cyclopropane carboxylique et de L'alcool (S) cyano [(3-phénoxy 5-fluoro) phényl] méthylique, on obtient le produit recherché F = 133¤C.

**Stade B :** 1R-[1-alpha-(S) 3-alpha-(R)] 3-[2,2,2-tribromo 1-hydroxy éthyl] 2,2-diméthyl cyclopropane carboxylate de (S) cyano [(3-phénoxy 4-fluoro) phényl] méthyle.

En opérant comme à l'exemple 7 par hydrolyse du produit obtenu au stade A, on obtient le produit recherché utilisé tel quel dans le stade suivant.

**Stade C :** 1R-[1-alpha-(S) 3-alpha-(R)] 2,2-diméthyl 3-[2,2,2-tribromo 1-méthylsulfonyloxyéthyl] cyclopropane carboxylate de (S) cyano [(3-phénoxy 4-fluoro) phényl] méthyle.

En opérant comme à l'exemple 7, à partir du produit obtenu au stade B du présent exemple et du chlorure de mésyle, on obtient le produit recherché.
$[alpha]_D$ = +34¤ ± 1¤ c = 1% $CHCl_3$

**Exemple 9 : 1R-[1-alpha-(S) 3-alpha-(R)] 2,2-diméthyl 3-[2,2,2-tribromo 1-(2-propène 1-sulfonyloxy) éthyl] cyclopropane carboxylate de cyano (3-phénoxyphényl) méthyle.**

En estérifiant le produit de l'exemple 1, par le chlorure de 1-sulfonyl oxy 2-propényle. On obtient le produit recherché fondant à 96¤C.

**Exemple 10 : 1R-[1-alpha-(S) 3-alpha-(R)] 2,2-diméthyl 3-[2,2,2-tribromo 1-trifluorométhyl carbonyloxyéthyl] cyclopropane carboxylate de (S) cyano (3-phénoxyphényl) méthyle.**

**Stade A :** 1R-[1-alpha, 3-alpha-(R)] 2,2-diméthyl 3-[2,2,2-tribromo 1-trifluorométhyl carbonyloxyéthyl] cyclopropane carboxylate de 1,1-diméthyléthyle.

On ajoute à 10/15¤C 16 cm3 d'acide trifluoroacétique dans un mélange de 5 g de 2,2-diméthyl 3-[2,2,2-tribromo 1-hydroxyéthyl cyclopropane carboxylate de terbutyle et de 20 cm3 de pyridine. On laisse remonter le mélange réactionnel à 20¤/25¤C et agite pendant 1h30. On verse le mélange réactionnel sur un mélange d'eau et de glace. On extrait à l'éther, sèche et amène à sec sous pression réduite. On refroidit à l'hexane, sèche et amène à sec le produit obtenu. On obtient 5,09g de produit recherché fondant à 102¤C.

**Stade B :** Acide 1R-[1-alpha, 3-alpha-(R)] 2,2-diméthyl 3-[2,2,2-tribromo 1-trifluorométhyl carbonyloxyéthyl] cyclopropane carboxylique.

En opérant comme au stade B de l'exemple à partir de l'acide obtenu au stade A du présent exemple, on obtient le produit recherché : F = 178¤C.

**Stade C :** 1R-[1-alpha-(S) 3-alpha-(R)] 2,2-diméthyl 3-[2,2,2-tribromo 1-trifluorométhyl carbonyloxyéthyl) cyclopropane carboxylate de (S) cyano (3-phénoxy phényl) méthyle.

En opérant comme au stade C de l'exemple à partir de l'acide obtenu au stade B du présent exemple et de l'alcool (S) alpha-cyano 3-phénoxy-benzylique, on obtient le produit recherché fondant à 79¤C.

**Exemple 11 : 1R-[1-alpha-(S)3-alpha-(R)] 2,2-diméthyl 3-[(1-diéthoxy phosphoryloxy) 2,2,2-tribromoéthyl] cyclopropane carboxylate de cyano 3-(phénoxyphényl) méthyle.**

**Stade A :** 1R-[1-alpha, 3-alpha-(R)] 3-[2,2,2-tribromo-(diéthoxy phosphoryloxy) éthyl] 2,2-diméthyl cyclopropane carboxylate de diméthyl éthyle.

On ajoute à -60¤C 5,05g de bromoforme, 35g de terbutylate de potasse et 20cm3 de tétrahydrofuran dans une suspension renfermant 4 g de céto [1-alpha, 3-alpha (R)] cyclopropane carboxylate de diméthyl éthyle et 40 cm3 de tétrahydrofuran. On agite pendant 15 minutes à -60¤C et ajoute 3,6g de chlorure de diéthoxyphosphoryloxy. On agite pendant 1h30 à -60¤C et verse dans une solution normale d'acide chlorhydrique. On extrait à l'éther, lave à l'eau, sèche et amène à sec sous pression réduite. On obtient

7,09g de produit recherché fondant à 79¤C.

**Stade B :** Acide 1R-[1-alpha, 3-alpha-(R)] 3-[2,2,2-tribromo 1-(diéthoxy phosphoryloxy) éthyl] 2,2-diméthyl cyclopropane carboxylique.

On porte au reflux pendant 1 heure une solution renfermant 5g de produit préparé au stade A, 50cm3 de benzène et 0,5g d'acide paratoluène sulfonique. On refroidit à 20/20¤C, le mélange réactionnel et le verse dans l'eau. On décante la phase organique, sèche et amène à sec sous pression réduite. On reprend le résidu à l'hexane et essore le produit obtenu. On obtient 3g d'un produit fondant à 146¤C.

**Stade C :** 1R-[1-alpha-(S) 3-alpha-(R)] 2,2-diméthyl 3-[(1-diéthoxy phosphoryloxy) 2,2,2-tribromoéthyl] cyclopropane carboxylate de cyano (3-phénoxyphényl) méthyle.

On introduit à 0 ± 5¤C, 1,22g de cyclohéxylcarbodiimide et 5 cm3 de chlorure de méthylène dans un mélange renfermant 3g d'acide préparé au stade B, 1,34g d'alcool (S) alpha-cyano 3-phénoxybenzylique, 50 mg de diméthylaminopyridine et 30 cm3 de chlorure de méthylène. On laisse le mélange réactionnel, revenir à la température ambiante et le maintient sous agitation pendant 1 h 30. On essore et amène à sec le produit obtenu. On chromatographie sur silice le produit obtenu en éluant par le mélange hexane-acétate d'éthyle 6-4. On obtient 3,3g de produit recherché.

**Exemple 12 : 1R-[1-alpha-(S)3-alpha-(R)] 3-[2,2,2-tribromo-1-(2-oxo 1,3,2-dioxaphospholannyloxy) éthyl] 2,2-diméthyl cyclopropane carboxylate de cyano (3-phénoxyphényl) méthyle.**

**Stade A :** 1R-[1-alpha, 3-alpha-(R)] 3-[2,2,2-tribromo 1-(2-oxo 1,3,2-dioxaphospholannyloxy) éthyl] 2,2-diméthyl cyclopropane carboxylate de 1,1-diméthyl éthyle.

On ajoute à -60¤C 4,6 cm3 de bromoforme dans une solution de 9,9g de 2,2-diméthyl 3-céto (1R, 3-alpha) cyclopropane carboxylate de 1,1-diméthyl éthyle dans 100 cm3 de tétrahydrofuran. On introduit ensuite à -60¤C 6,05g de terbutylate de potassium et 50 cm3 de tétrahydrofuran. On introduit ensuite en 15 minutes à -60¤C, 7,5g de chlorure de 2-oxo 1,3,2-dioxophospholane et 25 cm3 de tétrahydrofuran. On maintient sous agitation à -60¤C pendant 15 minutes. On verse sur une solution aqueuse d'acide chlorhydrique. On extrait au chlorure de méthylène, lave les phase organiques à l'eau, sèche et amène à sec sous pression réduite. On obtient 27g d'un produit que l'on empâte avec 100 cm3 d'éther éthylique. On filtre et sèche le produit obtenu. On obtient 18,4g de produit recherché fondant à 210¤C.

**Stade B :** Acide 1R-[1-alpha, 3-alpha-(R)] 3-[2,2,2-tribromo-1- (2-oxo 1,3,2-dioxaphospholannyloxy) éthyl] 2,2-diméthyl cyclopropane carboxylique.

On introduit 12,9g de produit préparé au stade A, au reflux d'une solution de 500 cm3 de benzène et d'acide paratoluène sulfonique. On maintient au reflux pendant 30 minutes. On amène à sec le produit obtenu, le reprend à l'acétate d'éthyle, agite 15 minutes et filtre. On obtient 5,5g du produit recherché fondant à 225¤C.

**Stade C :** 1R-[1-alpha-(S) 3-alpha-(R)] 3-[2,2,2-tribromo 1-(2-oxo 1,3,2-oxaphospholannyloxy) éthyl] 2,2-diméthyl cyclopropane carboxylate de (S) cyano (3-phénoxyphényl) méthyle.

En opérant comme à l'exemple 1, stade B, à partir de 2,5 g du produit obtenu ci-dessus et 1,07 g d'alcool (S) alpha cyano 3-phénoxy benzylique, on a préparé le produit attendu. F = 155¤C.
$[alpha]_D$ = +42¤ ± 1,5¤ (c = 1%, $CHCl_3$).

**Exemple 13 : 1R-[1-alpha, 3-alpha-(R)] 3-[2,2,2-tribromo 1-(2-oxo 1,3,2-dioxaphospholannyloxy) éthyl] 2,2 diméthyl cyclopropane carboxylate de (2-méthyl 3-phényl phényl) méthyle.**

A partir de l'acide préparé au stade B de l'exemple 6 et de l'alcool (2-méthyl 3-phénylphényl) méthylique, on obtient le produit recherché. F = 182¤C.

**Exemple 14 : 1R-[1-alpha-(S) 3-alpha-(R ou S)] 3-[2,2,2-trichloro-1-(diéthoxythiophosphoryloxy) éthyl] 2,2-diméthyl cyclopropane carboxylate de cyano (3-phénoxy phényl) méthyle.**

**Stade A :** 1R-[1-alpha, 3-alpha-(R+S)] 3-[2,2,2-trichloro-1-diéthoxy phosphoryloxy) éthyl] 2,2-diméthyl cyclopropane carboxylate de diméthyl éthyle.

On introduit à -60¤C 0,84cm3 de chloroforme et 1,21g de terbutylate de potassium dans 10cm3 de tétrahydrofuran dans une solution renfermant 1,98g de 2,2-diméthyl 3-céto (1-alpha, 3-alpha) cyclopropane carboxylate de 1,1-diméthyl éthyle dans 10 cm3 de tétrahydrofuran. On agite trente minutes à -60¤C et ajoute 1,88g de chlorothiophosphate de 0,0diéthyle. On laisse la température remonter à 20/25¤C. On agite pendant 5 heures. On verse le mélange réactionnel dans une solution normale d'acide chlorhydrique, extrait à l'éther, sèche et amène à sec sous pression réduite. On chromatographie sur silice en éluant par le mélange hexane-éther isopropylique 9-1. On obtient ainsi 2,15g de produit recherché.

**Stade B :** Acide 1R-[1-alpha, 3-alpha-(R+S)] 3-[2,2,2-trichloro-1-(diéthoxy thiophosphoryloxy) éthyl] 2,2-diméthyl cyclopropane carboxylique.

En opérant comme au stade B de l'exemple précédent, à partir du produit obtenu au stade A du présent exemple, on obtient le produit recherché.

**Stade C :** 1R-[1-alpha-(S) 3-alpha-(R ou S)] 3-[2,2,2-trichloro-1-diéthoxythiophosphoryloxy) éthyl] 2,2-diméthyl cyclopropane carboxylate de cyano (3-phénoxy phényl) méthyle.

En opérant comme au stade C de l'exemple précédent, à partir du produit obtenu au stade B du présent exemple, on obtient un produit que l'on chromatographie sur silice en éluant par le mélange hexane-acétate d'éthyle 7-3. On obtient 0,850 g d'isomère A.

**Spectre RMN**

$CH_3$ gem. 73-74Hz
H cyclopropyl 110 à 116Hz

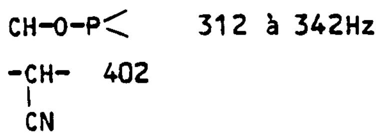


Aromatiques 416 à 457
et 0,800 g de produit B.

**Spectre RMN**

$CH_3$ gem. 73-75Hz

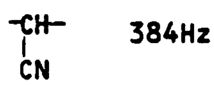


Aromatiques 425 à 452Hz

**Example 15 : 1R-[1-alpha, 3-alpha-(R)] 3-[2,2,2-trichloro 1-méthylsulfonyloxy éthyl] 2,2-diméthyl cyclopropane carboxylate de cyano-(3-phénoxy phényl) méthyle.**

**Stade A :** 1R-[1-alpha, 3-alpha-(R)] 3-[2,2,2-trichloro 1-hydroxy éthyl] 2,2-diméthyl cyclopropane carboxylate de diméthyl éthyle.

On introduit à -60, -55¤C sous agitation et atmosphère d'azote dans un mélange de 4,95g de 3-formyl 1R,3R 2,2-diméthyl cyclopropane carboxylate de terbutyle et 50 cm3 de tétrahydrofuranne 2,2 cm3 de chloroforme puis 2,95g de terbutylate de potassium et 25 cm3 de tétrahydrofuranne. On maintient le mélange réactionnel sous agitation pendant 2 h 30. On extrait à l'éther, on lave la phase organique, la

sèche et l'amène à sec sous pression réduite. On chromatographie le produit obtenu en éluant par le mélange hexane-acétate d'éthyle (7-1). On obtient ainsi 6,45g de produit. F = 50¤C.

**Stade B :** 1R-[1-alpha, 3-alpha-(R)] 3-[2,2,2-trichloro 1-méthyl sulfonyloxyéthyl] cyclopropane carboxylate de diméthyléthyle.

On ajoute à 0-5¤C dans un mélange de 6,35g de produit préparé au stade A, 65 cm3 de tétrahydrofuranne et 3,2 cm3 de chlorure de mésyle, 5,6 cm3 de triéthylamine et 6 cm3 de tétrahydrofuranne. On verse le mélange réactionnel dans l'eau. On extrait à l'éther, sèche sur sulfate de sodium, filtre et amène à sec. On obtient 8,1g de produit fondant à 106¤C.

**Stade C :** Acide 1R-(1-alpha, 3-alpha-(R)] 3-[2,2,2-trichloro 1-méthyl sulfonyloxy éthyl] cyclopropane carboxylique.

On porte au reflux pendant 1 heure 6,5g de produit préparé au stade précédent, 65 cm3 de benzène et 150 mg d'acide paratoluène sulfonique. On verse le mélange réactionnel dans l'eau, extrait au chlorure de méthylène et amène à sec. On obtient 5,15g de cristaux fondant à 148¤C.

**Stade D :**1R-[1-alpha, 3-alpha-(R)] 3[-2,2,2-trichloro 1-méthyl sulfonyloxy éthyl) 2,2-diméthyl cyclopropane carboxylate de cyano (3-phénoxy phényl) méthyle.

En opérant comme à l'exemple 1, en estérifiant l'acide obtenu au stade précédent par l'alcool (S) alpha-cyano 3-phénoxy benzylique, on obtient le produit recherché.
$[alpha]_D$ = +33¤ ± 1¤, c = 1% $CHCl_3$

**Exemple 16 : 1R-[1-alpha-(S) 3-alpha-(R)] 3-(2,2,2-trichloro 1-méthyl sulfonyloxy éthyl) 2,2-diméthyl cyclopropane carboxylate de (S) cyano (3-phénoxy 4-fluoro) phényl méthyle.**

Le produit a été préparé par estérification de l'acide obtenu au stade C de l'exemple 15 à l'aide d'alcool S-alpha cyano 4-fluoro 3-phénoxy benzylique.
$[alpha]_D$ = +38¤ ± 1¤, c = 1,5% $CHCl_3$.

**Exemple 17 : 1R-[1-alpha, 3-alpha-(R)] 3-[2,2,2-trichloro 1-méthyl sulfonyloxy éthyl] 2,2-diméthyl cyclopropane carboxylate de cyano 1-(6-phénoxy 2-pyridyl) méthyle.**

Le produit a été préparé par estérification de l'acide obtenu au stade C de l'exemple 15 à l'aide d'alcool RS-cyano 1-(6-phénoxy 2-pyridyl) méthylique.
$[alpha]_D$ = 2¤ ± 1¤, c= 0,8% $CHCl_3$.

**Exemple 18 : 1R-[1-alpha, 3-alpha-(R)] 3-[2,2,2-trichloro 1-méthyl sulfonyloxy éthyl] cyclopropane carboxylate de (2-méthyl 3-phényl phényl) méthyle.**

Le produit a été préparé par estérification de l'acide obtenu au stade C de l'exemple 15 à l'aide de l'alcool (2-méthyl 3-phényl benzylique).
$[alpha]_D$ = 20¤ ± 1¤, c = 1% $CHCl_3$

**Exemple 19 : 1R-[1-alpha-(S*) 3-alpha-(R*)] 2,2-diméthyl 3-[2,2,2-trichloro 1-(4-méthylphényl sulfonyloxy) éthyl] cyclopropane carboxylate de cyano (3-phénoxy phényl) méthyle.**

**Stade A :** 1R-[1-alpha, 3-alpha-(R*)] 3-[2,2,2-trichloro 1-(4-méthyl phényl) sulfonyloxy] éthyl 2,2-diméthyl cyclopropane carboxylate de diméthyl éthyle.

On introduit à -60¤C 1,7 cm3 de chloroforme, puis 2,42g de terbutylate de potassium et 20 cm3 de tétrahydrofuranne dans une suspension de 3,96g de de (1R,3R) 3-formyl cyclopropane carboxylate de terbutyle et 20 cm3 de tétrahydrofuranne. On agite pendant 30 minutes à -60¤C. On introduit en 15 minutes à -60¤C, 4g de chlorure de tosyle et 10 cm3 de tétrahydrofuranne. On agite pendant 30 minutes à -60¤C et verse dans 200 cm3 d'une solution normale d'acide chlorhydrique normal. On extrait à l'éther sulfurique, lave à l'eau, sèche et amène à sec sous pression réduite. On obtient ainsi 5,3g de produit

recherché.

**Stade B :** Acide 1R-[1-alpha, 3-alpha-(R*)] 3-[2,2,2-trichloro 1-(4-méthylphényl sulfonyloxy) éthyl] 2,2-diméthyl cyclopropane carboxylique.

Il est obtenu par chauffage au reflux du benzène de l'ester précédent en présence d'acide paratoluène sulfonique. F = 190¤.

**Stade C :** 1R-[1-alpha-(S*) 3-alpha-(R*)] 2,2-diméthyl 3-[2,2,2-trichloro 1(4-méthylphényl sulfonyloxy) éthyl] cyclopropane carboxylate de cyano (3-phénoxy phényl) méthyle.

L'estérification avec l'alcool S-alpha cyano 3-phénoxy benzylique est réalisée dans les conditions du stade C de l'exemple 1. On a obtenu le produit recherché.
$[alpha]_D$ = 77,5¤ ± 1,5¤ c = 1,1% $CHCl_3$

**Exemple 20 : 1R-[1-alpha-(S) 3-alpha-(S)] 2,2-diméthyl 3-[2,2,2-tribromo 1-méthyl sulfonyloxy éthyl] cyclopropane carboxylate de cyano (3-phénoxy phényl) méthyle.**

**Stade A :** 1R-[1-alpha, 3-alpha-(S)] 2,2-diméthyl 3-[2,2,2-tribromo 1-hydroxy éthyl] cyclopropane carboxylate de 1,1 diméthyle éthyle.

On ajoute 26 cm3 de bromoforme à 39,6g de 1R, 3R, 2,2-diméthyl 3-céto cyclopropane de 1,1-diméthyl éthyle en solution dans 200 cm3 de tétrahydrofuranne. On ajoute à la solution obtenue 17,5g de méthylate de potassium 100 cm3 de terbulonal, 60 cm3 de tétrahydrofuranne et 60 cm3 de diméthylformamide. On agite 1 heure à -10¤C, puis 1 heure à +10¤C. On verse dans l'eau. On extrait à l'éther isopropylique. On sèche et amène à sec, sous vide. On sépare par chromatographie les deux diastéréoisomères obtenus.

**Stade B :** 1R-[1-alpha, 3-alpha-(S)] 2,2-diméthyl 3-[2,2,2-tribromo 1-méthyl sulfonyloxy éthyl cyclopropane carboxylate de 1,1-diméthyl éthyle.

On refroidit à 0¤ ± 5¤ un mélange renfermant 4g de produit préparé au stade précédent, 40 cm3 de tétrahydrofuranne et 2,03g de chlorure de mésyle. On ajoute à 0¤ ± 5¤C 1,78g de triéthylamine et 5 cm3 de tétrahydrofuranne. On agite pendant 2 heures à 0¤ ± 5¤C. On verse dans l'eau, extrait au chlorure de méthylène, sèche et amène à sec sous pression réduite. On obtient un produit que l'on purifie par recristallisation dans l'hexane, puis dans l'éther isopropylique. On obtient 2,9g de produit fondant à 159¤C.

**Stade C :** Acide 1R-[1-alpha, 3-alpha-(S)] 2,2-diméthyl 3-[2,2,2-tribromo 1-méthylsulfonyloxy éthyl] cyclopropane carboxylique.

On porte au reflux un mélange de 2,3g de de produit préparé au stade B et 25 cm3 de benzène. On ajoute 70mg d'acide paratoluène sulfonique. On refroidit, lave à l'eau, sèche et amène à sec sous pression réduite. On obtient 1,8g de produit recherché fondant à 168¤C.

**Stade D :** 1R-[1-alpha (S) 3-alpha (S)] 2,2-diméthyl 3-[2,2,2-tribromo 1-méthyl sulfonyloxy] cyclopropane carboxylate de cyano (3-phénoxyphényl) méthyle.

On introduit 0,78 g de dicyclohexylcarbodiimide et 3 cm3 de chlorure de méthylène dans une solution renfermant 1,8 g de l'acide obtenu ci-dessus alpha alpha, 20 cm3 de chlorure de méthylène, 0,86 g d'alcool (S) alphacyano 3-phénoxy benzylique et 0,1 g de diméthylaminopyridine. On agite à 20-25¤C pendant 2 heures. On essore le produit formé et amène à sec sous pression réduite le filtrat. On effectue une chromatograpie en éluant par le mélange hexane-acétate d'éthyle 7-3. On obtient ainsi 2 g de produit recherché.
$[alpha]_D$ = 1¤ ± 1¤, c = 1%, $CHCl_3$.

**Exemple 21 : 1R [1-alpha (S) 3-alpha (R)] 2,2-diméthyl 3-[2,2,2-tribromo 1-[(3-hydroxy 2-hydroxy 2-méthyl) propylsulfonyloxy) éthyl] cyclopropane carboxylate de cyano (3-phénoxyphényl) méthyle.**

On mélange à température ambiante 5 g de produit obtenu à l'exemple 5 et 3,75 g de N-méthylmorpholine N-oxyde dans 125 cm3 de tétrahydrofuranne et 40 cm3 d'eau. On ajoute 100 mg de tétroxyde d'osmium, agite 22 heures à 20¤C, dilue le milieu réactionnel avec 250 cm3 de chlorure de méthylène, refroidit à 0¤C puis ajoute lentement 6 g d'hydrosulfite de sodium en solution dans 40 cm3 d'eau. On laisse revenir à température ambiante, sèche et élimine les solvants sous pression réduite. On obtient 5,1 g de produit brut que l'on chromatographie sur silice (éluant : hexane-acétate d'éthyle (85-15). On recueille 1,7 g de produit attendu, puis après traitement à l'éther, 1,4 g de produit amorphe.
$[alpha]_D$ = +38¤ ± 1¤ (c = 1%, chloroforme).

| Analyse : $C_{26}H_{28}Br_3NO_8S$ : 754,306 | | | | | |
|---|---|---|---|---|---|
| Calculé : | C% 41,40 | H% 3,74 | N% 1,86 | Br% 31,78 | S% 4,25 |
| Trouvé : | 41,7 | 3,7 | 1,9 | 30,0 | 4,1 |

**Exemple 22 : 1R [1-alpha (S) 3-alpha (R)] 2,2-diméthyl 3-[2,2,2-tribromo 1-[(3-hydroxy 2-hydroxy) propylsulfonylloxy) éthyl] cyclopropane carboxylate de cyano (3-phénoxyphényl) méthyle.**

On opère comme à l'exemple 21 à partir du produit obtenu à l'exemple 9. Après chromatographie sur silice (éluant : chlorure de méthylène-acétate d'éthyle 1-1) puis reprise du résidu dans l'éther, on obtient 2,6 g de produit attendu.
$[alpha]_D$ = +41¤ ± 1,5¤ (c = 0,7%, $CHCl_3$).

| Analyse : $C_{25}H_{26}Br_3NO_8S$ : 754,306 | | | | | |
|---|---|---|---|---|---|
| Calculé : | C% 40,56 | H% 3,54 | N% 1,89 | Br% 32,38 | S% 4,33 |
| Trouvé : | 40,8 | 3,3 | 1,8 | 30,5 | 4,3 |

**Exemple 23 : 1R [1-alpha (S) 3-alpha (R)] 2,2-diméthyl 3-[2,2,2-tribromo 1-[(2,2-diméthyl 1,3-dioxolan-4-yl) méthylsulfonyloxy) éthyl] cyclopropane carboxylate de cyano (3-phénoxyphényl) méthyle.**

On agite 2 heures à 20¤C, 1,3 g du produit obtenu à l'exemple 22 et 130 mg d'acide paratoluène sulfonique dans 130 cm3 d'acétone, dilue avec te 100 cm3 d'eau, sèche et élimine les solvants sous pression réduite. On recueille 1,25 g de produit brut que l'on chromatographie sur silice (éluant : hexane-acétate d'éthyle 3-1) et obtient 1,15 g de produit attendu.
$[alpha]_D$ = =44¤ ± 1,5¤ (c = 1%, $CHCl_3$).

En opérant comme indiqué dans les exemples précédents, on a préparé les produits suivants :

**Exemple 24 : (1R,cis) 2,2-diméthyl 3-(2,2,2-trichloro 1R-acétyloxyéthyl) cyclopropane carboxylate de (3-phénoxyphényl) méthyle.**

| Analyse : $C_{23}H_{23}Cl_3O_5$ | | | |
|---|---|---|---|
| Calculé : | C% 56,87 | H% 4,77 | Cl% 21,89 |
| Trouvé : | 56,9 | 4,8 | 21,7 |

**Exemple 25 : (1R,cis) 2,2-diméthyl 3-(2,2,2-trichloro 1R-méthanesulfonyl oxyéthyl) cyclopropane carboxylate de (R) ou (S) cyano (3-phénoxyphényl) méthyle.**
$[alpha]_D$ = +7¤ (c = 0,75% benzène.

**Exemple 26 : préparation d'un concentré soluble.**

On effectue un mélange homogène de :

| | |
|---|---|
| Produit de l'exemple 2 | 0,25 g |
| Butoxyde de pipéronyle | 1,00 g |
| Tween 80 | 0,25 g |
| Topanol A | 0,1 g |
| Eau | 98,4 g |

**Exemple 27 : préparation d'un concentré émulsifiable.**

On mélange intimement :

| | |
|---|---|
| Produit de l'exemple 3 | 0,015 g |
| Butoxyde de pipéronyl | 0,5 g |
| Topanol A | 0,1 g |
| Tween 80 | 3,5 g |
| Xylène | 95,885 g |

**Exemple 28 : préparation d'un concentré émulsifiable.**

On effectue un mélange homogène de :

| | |
|---|---|
| Produit de l'exemple 8 | 1,5 g |
| Tween 80 | 20,00 g |
| Topanol A | 0,1 g |
| Xylène | 78,4 g |

**Exemple 29 : préparation d'une composition fumigène.**

On mélange d'une façon homogène :

| | |
|---|---|
| Produit de l'exemple 20 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-nitrophénol | 0,5 g |

**Exemple 30 : exemple d'aliment composé pour animaux.**

On utilise comme aliment équilibré de base un aliment comportant du maïs, de la luzerne déshydratée, de la paille de blé, du tourteau de palmiste mélassé, de l'urée, un condiment minéral vitaminé.

Cet aliment contient au minimum 11% de matières protéiques brutes (dont 2,8% apportés par l'urée), 2,5% de matières grasses et au maximum 15% de matières cellulosiques, 6% de matières minérales et 13% d'humidité.

L'aliment utilisé correspond à 82 unités fourragères pour 100 kilos et contient pour 100 kilos 910.000 U.I. de vitamine A, 91.000 U.I. de vitamines $D_3$, 150mg de vitamine E, 150mg de vitamine C.

On incorpore à cet aliment 0,3kg de composé ce l'exemple 1 pour 100 kg d'aliment au total.

**Exemple 31 : Exemple d'aliment composé pour animaux.**

On utilise le même aliment équilibré de base qu'à l'exemple 44. On incorpore à cet aliment, 0,04kg de

composé de l'exemple 2 pour 100kg d'aliment au total.

ETUDE BIOLOGIQUE

1)Etude de l'activité de choc sur mouche domestique

Les insectes tests sont des mouches domestiques femelles âgées de 4 à 5 jours. On opère par pulvérisation topique de 1 microlitre de solution acétonique sur le thorax dorsal des insectes à l'aide du micromanipulation d'Arnold.

On utilise 50 individus par traitement. On effectue le contrôle de mortalité 24 heures après traitement.

Les résultats obtenus exprimés en DL 50 ou dose en nanogrammes par individu nécessaire pour tuer 50% des insectes, sont les suivants :

| Composé de l'exemple | DL 50 ng/insecte |
|---|---|
| 2 | 1,7 |
| 3 | 2,7 |
| 4 | 8,7 |
| 8 | 1,3 |
| 20 | 2,3 |
| 21 | 5,7 |
| 22 | 5,2 |
| 23 | 4,5 |

Conclusion : Les produits de l'invention sont doués d'un très bon effet de choc sur mouches domestiques.

2) Etude de l'activité par contact tarsal sur blatte germanique.

Les insectes testés sont des mâles de blatte germanique (Blatella germanica). On opère par dépôt d'une solution acétonique de concentration déterminée sur le fond d'une boîte de Petri de 20 cm de diamètre. Après séchage, on laisse séjourner 20 mâles de blattes par concentration durant 1 heure puis on transfère les insectes sur milieu sain et on contrôle leur mortalité à 24h, 48h, 3 et 5 jours.

Le résultat est exprimé en concentration létale 50 (CL 50) en mg/m2.

| Exemple | CL 50 (mg/m2) |
|---|---|
| 2 | 1,2 |
| 3 | 0,2 |
| 4 | 2 |
| 5 | 2,5 |
| 8 | 0,07 |
| 9 | 0,06 |
| 20 | 0,3 |
| 21 | 0,30 |
| 22 | 0,29 |
| 23 | 0,22 |

3) Etude de l'effet létal sur larves de Spodoptera Littoralis.

Les essais sont effectués par application topique d'une solution acétonique à l'aide du micro manipulateur d'Arnold sur le thorax dorsal des larves. Onutilise 15 larves par dose de produit à tester. Les larves utilisées sont des larves du quatrième stade larvaire, c'est-à-dire âgées d'environ 10 jours lorsqu'elles sont élevées à 24¤C et 65% d'humidité relative. Après traitement, les individus sont placés sur un milieu nutritif artificiel (milieu de Poitout).

On effectue le contrôle des mortalités 48 heures après traitement.

Les résultats expérimentaux obtenus sont résumés dans le tableau suivant :

Etude de l'activité par application topique sur larve de spodoptera littoralis.

| Test Résultats | |
|---|---|
| Exemple | DL 50 en (ng/insecte) |
| 2 | 13 |
| 3 | 11 |
| 4 | 25 |
| 8 | 2,8 |
| 20 | 6,5 |

**Revendications**
**Revendications pour les Etats contractants suivants : AT, BE, CH, DE, FR, GB, GR, IT, LI, LU, NL**

**1.** Les composés de formule (I) :

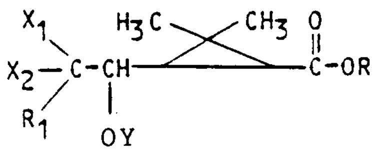

(I)

dans laquelle $X_1$ et $X_2$ identiques ou différents l'un de l'autre représentent un atome d'halogène, $R_1$ représente un atome d'halogène, un radical alkyle renfermant jusqu'à 8 atomes de carbone, un radical aryle renfermant jusqu'à 14 atomes de carbone éventuellement substitué, un radical perfluoroalkyle renfermant jusqu'à 8 atomes de carbone, un radical -C≡N, un radical

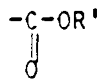

dans laquelle R' représente un radical alkyle renfermant jusqu'à 8 atomes de carbone, Y représente un radical choisi dans le groupe constitué par :

- les radicaux $SO_2alc_1$, $alc_1$ représentant un radical alcoyle linéaire ou ramifié, saturé ou insaturé éventuellement substitué par un ou plusieurs groupements fonctionnels ;
- les radicaux $SO_2A_r$, $A_r$ représentant un radical aryle renfermant jusqu'à 14 atomes de carbone, éventuellement substitué par un ou plusieurs groupements fonctionnels ;
- les radicaux

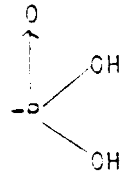

ou

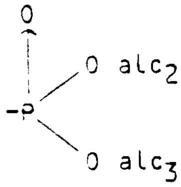

$alc_2$ et $alc_3$ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou $alc_2$ et $alc_3$ pouvant former avec le radical

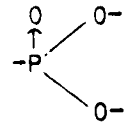

,

un cycle

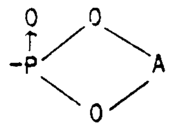

A représentant une chaîne carbonée linéaire ou ramifiée, saturée ou insaturée éventuellement substituée par un ou plusieurs groupements fonctionnels et renfermant jusqu'à 6 atomes de carbone ;

- les radicaux

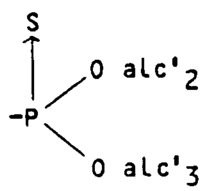

,

alc'$_2$ et alc'$_3$ représentant les mêmes valeurs que alc$_2$ et alc$_3$ ;

- le radical

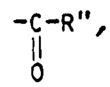

R" représentant un radical aryle et R représente

- soit un atome d'hydrogène,
- soit un radical alkyle, linéaire ou ramifié, éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

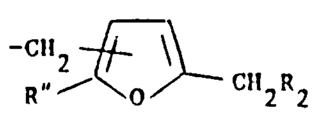

  dans lequel le substituant R" représente un atome d'hydrogène ou un radical méthyle et le substituant R$_2$ un aryle monocyclique ou un groupement -C≡CH et notamment un groupement 5-benzyl 3-furyl méthyle,
- soit un groupement :

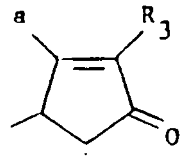

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- soit un groupement :

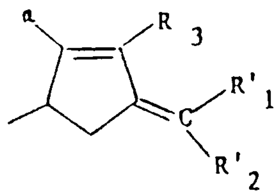

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

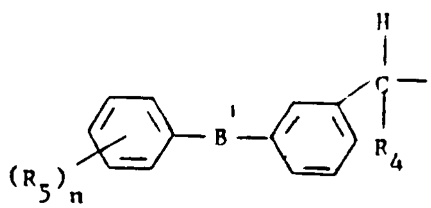

dans lequel B' représente un atome d'oxygène ou de soufre, un groupement

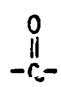

ou $-CH_2$ ou un groupement sulfoxyde ou un groupement sulfone et $R_4$ représente un atome d'hydrogène, un radical $-C\equiv N$, un radical méthyle, un radical $-CONH_2$, un radical $-CSNH_2$ ou un radical $-C\equiv CH$, $R_5$ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,

- soit un groupement :

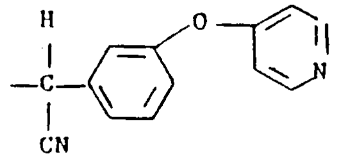

- soit un groupement :

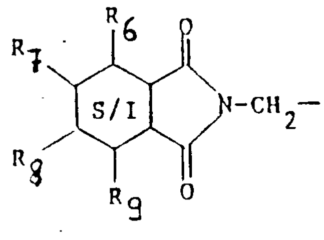

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

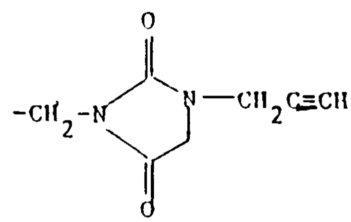

- soit un groupement :

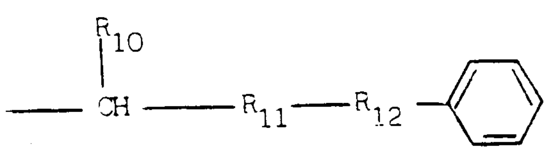

dans lequel $R_{10}$ représente un atome d'hydrogène ou un radical CN, $R_{12}$ représente un radical $-CH_2$ ou un atome d'oxygène, $R_{11}$ représente un radical thiazolyle ou thiadiazolyle dont la liaison avec

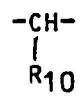

peut se trouver à l'une quelconque des positions disponibles, $R_{12}$ étant lié à $R_{11}$ par l'atome de carbone compris entre l'atome de soufre et un atome d'azote,

- soit un groupement :

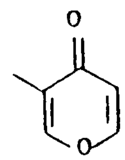

- soit un groupement :

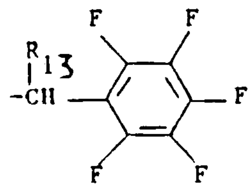

dans lequel $R_{13}$ représente un atome d'hydrogène ou un radical CN,
- soit un groupement :

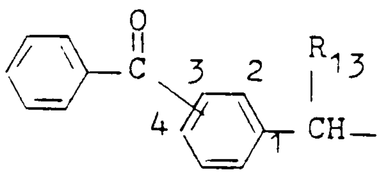

dans lequel $R_{13}$ est défini comme ci-dessus, et le radical benzoyle est en position 3 ou 4,
- soit un groupement :

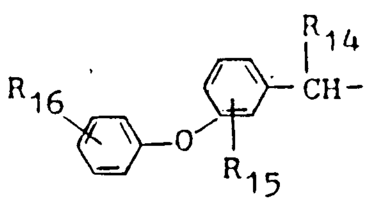

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,
- soit un groupement :

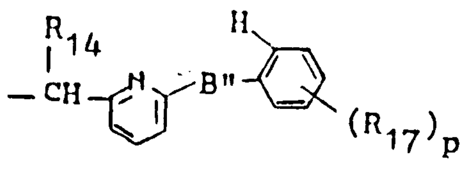

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et B" représente un atome d'oxygène ou un atome de soufre,
- soit un groupement :

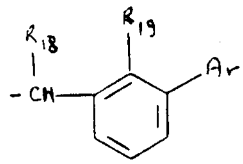

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,

- soit un groupement :

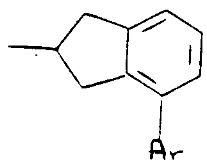

ou

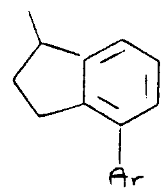

- soit un groupement :

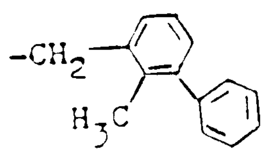

- soit un groupement :

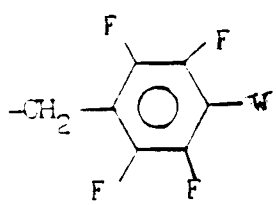

avec W=H, $CH_3$, $OCH_3$

à la condition que $R_1$ ne représente pas un radical $CF_3$ si R = H alkyle.

**2.** Les composés de formule tels que définis à la revendication 1 dans lesquels R représente un radical :

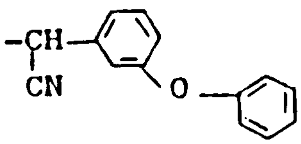

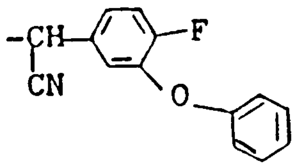

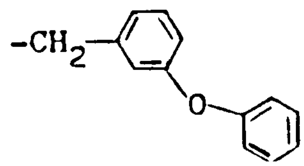

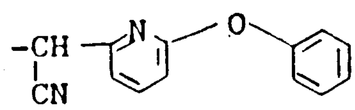

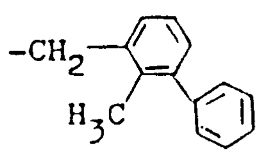

ou 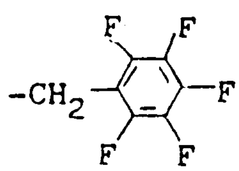 ou 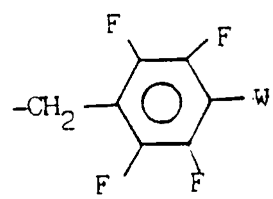

avec W=H, $CH_3$, $OCH_3$

ou 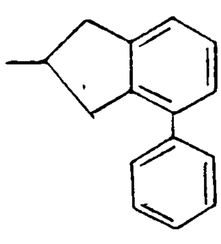

**3.** Les composés de formule I tels que définis à la revendication 1 ou 2 dans lesquels $X_1$ et $X_2$ représentent un atome de chlore, de brome ou d'iode.

**4.** Les composés de formule I tels que définis à la revendication 3 dans lesquels $X_1$ et $X_2$ représentent un atome de brome.

**5.** Les composés de formule I tels que définis à l'une quelconque des revendications 1 à 4 dans lesquels $R_1$ représente un atome de brome, de chlore ou de fluor.

**6.** Les composés de formule I tels que définis à l'une quelconque des revendications 1 à 5 dans lesquels Y représente un radical $SO_2alc_1$, $alc_1$ représentant un radical alkyle ou alkényle renfermant jusqu'à 8 atomes de carbone.

**7.** Les composés de formule I tels que définis à la revendication 6 dans lesquels Y représente un radical $SO_2CH_3$.

**8.** Les composés de formule I tels que définis à l'une quelconque des revendications 1 à 5 dans lesquels Y représente un radical $SO_2Ar$, Ar conservant la même signification que précédemment.

**9.** Les composés de formule I tels que définis à la revendication 1 dont les noms suivent :
- le 1R-[1-alpha-(S) 3-alpha-(R)] 2,2-diméthyl 3-[1-(méthylsulfonyloxy) 2,2,2-tribromoéthyl] cyclopropane carboxylate de cyano (3-phénoxy-phényl) méthyle,
- le 1R-[1-alpha-(S) 3-alpha-(R)] 2,2-diméthyl 3-[2,2,2 tribromo 1-méthyl sulfonyloxy éthyl] cyclopropane carboxylate de cyano [(3-phénoxy 4-fluoro)phényl] méthyle,
- le 1R-[1-alpha, 3-alpha (RS*,RS*)] 2,2-diméthyl 3-[2-trifluorométhyl 2-bromo 2-chloro 1-méthyl sulfonyloxy éthyl] cyclopropane carboxylate de cyano (3-phénoxyphényl) méthyle.

**10.** Procédé de préparation des composés de formule I de la revendication 1, dans laquelle $X_1$, $X_2$ et R conservent la même signification que dans la revendication 1, $R_1$ conserve la même signification que dans la revendication 1 et peut en outre représenter un radical perfluoroalkyle renfermant jusqu'à 8 atomes de carbone, Y conserve la même signification que dans la revendication 1 et peut en outre représenter un radical

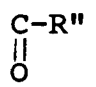

dans laquelle R" représente un radical alkyle éventuellement substitué caractérisé en ce que l'on soumet un composé de formule :

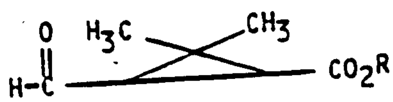

(II)

dans laquelle R conserve la même signification que précédemment à l'action d'un composé de formule V :

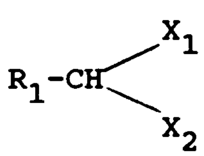

(V)

dans laquelle $X_1$, $X_2$ et $R_1$ conservent la même signification que précédemment en présence d'une base pour obtenir le composé de formule IV :

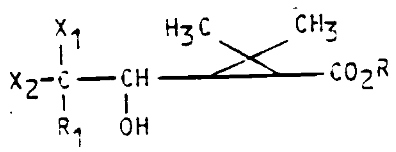

(IV)

que l'on soumet à l'action d'un agent de sulfonylation ou de phosphonylation ou de thiophosphorylation ou d'acylation pour obtenir le composé de formule I correspondant :

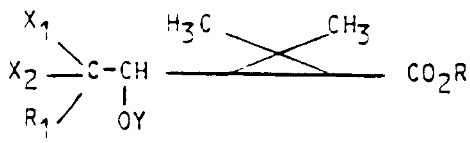

que l'on transforme éventuellement en acide correspondant que l'on soumet éventuellement à un agent d'estérification pour obtenir un nouveau composé de formule I.

**Revendications pour l'Etat contractant suivant : ES**

**1.** Procédé de préparation des composés de formule (I) :

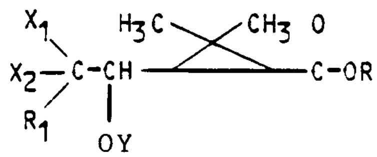

(I)

dans laquelle $X_1$ et $X_2$ identiques ou différents l'un ce l'autre représentent un atome d'halogène, $R_1$ représente un atome d'halogène, un radical alkyle renfermant jusqu'à 8 atomes de carbone, un radical aryle renfermant jusqu'à 14 atomes de carbone éventuellement substitué, un radical perfluoroalkyle renfermant jusqu'à 8 atomes de carbone, un radical -C≡N, un radical

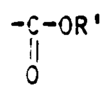

dans laquelle R' représente un radical alkyle renfermant jusqu'à 8 atomes de carbone, Y représente un radical choisi dans le groupe constitué par :

- les radicaux $SO_2alc_1$, $alc_1$ représentant un radical alcoyle linéaire ou ramifié, saturé ou insaturé éventuellement substitué par un ou plusieurs groupements fonctionnels ;
- les radicaux $SO_2A_r$, $A_r$ représentant un radical aryle renfermant jusqu'à 14 atomes de carbone, éventuellement substitué par un ou plusieurs groupements fonctionnels ;
- les radicaux

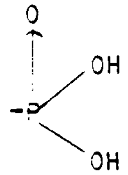

ou

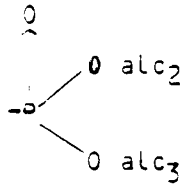

$alc_2$ et $alc_3$ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou $alc_2$ et $alc_3$
pouvant former avec le radical

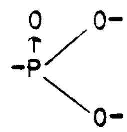

,

un cycle

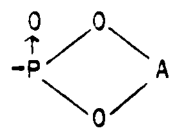

A représentant une chaîne carbonée linéaire ou ramifiée, saturée ou insaturée éventuellement substituée par un ou plusieurs groupements fonctionnels et renfermant jusqu'à 6 atomes de carbone ;

- les radicaux

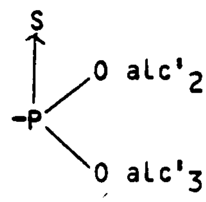

,

$alc'_2$ et $alc'_3$ représentant les mêmes valeurs que $alc_2$ et $alc_3$ ;

- le radical

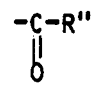

,

R" représentant un radical aryle et
R représente

- soit un atome d'hydrogène,
- soit un radical alkyle, linéaire ou ramifié, éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

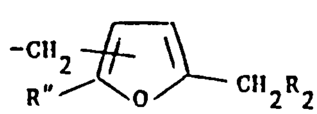

dans lequel le substituant R" représente un atome d'hydrogène ou un radical méthyle et le

substituant $R_2$ un aryle monocyclique ou un groupement -C≡CH et notamment un groupement 5-benzyl 3-furyl méthyle,

- soit un groupement :

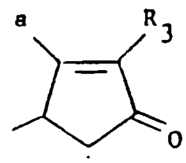

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C≡CH$,

- soit un groupement :

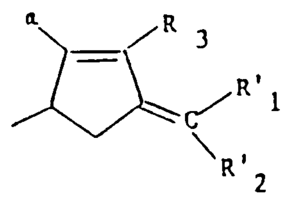

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

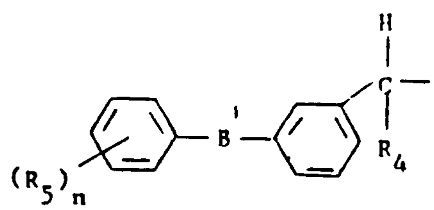

dans lequel B' représente un atome d'oxygène ou de soufre, un groupement

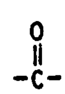

ou $-CH_2$ ou un groupement sulfoxyde ou un groupement sulfone et $R_4$ représente un atome d'hydrogène, un radical -C≡N, un radical méthyle, un radical $-CONH_2$, un radical $-CSNH_2$ ou un radical -C≡CH, $R_5$ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,

- soit un groupement :

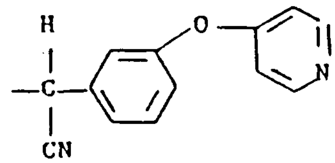

- soit un groupement :

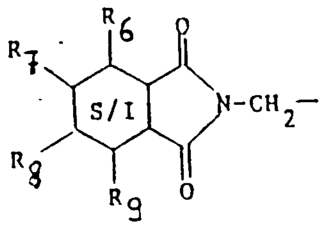

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

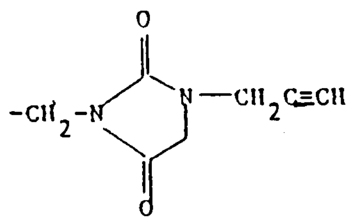

- soit un groupement :

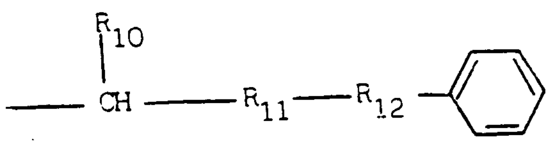

dans lequel $R_{10}$ représente un atome d'hydrogène ou un radical CN, $R_{12}$ représente un radical $-CH_2$ ou un atome d'oxygène, $R_{11}$ représente un radical thiazolyle ou thiadiazolyle dont la liaison avec

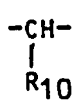

peut se trouver à l'une quelconque des positions disponibles, $R_{12}$ étant lié à $R_{11}$ par l'atome de carbone compris entre l'atome de soufre et un atome d'azote,

- soit un groupement :

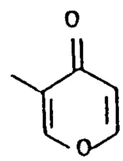

- soit un groupement :

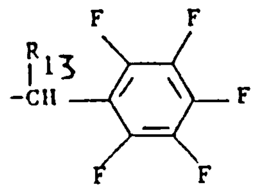

dans lequel $R_{13}$ représente un atome d'hydrogène ou un radical CN,

- soit un groupement :

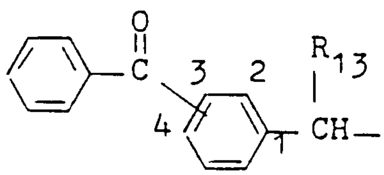

dans lequel $R_{13}$ est défini comme ci-dessus, et le radical benzoyle est en position 3 ou 4,

- soit un groupement :

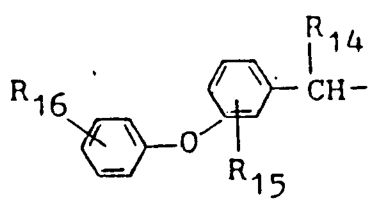

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupement :

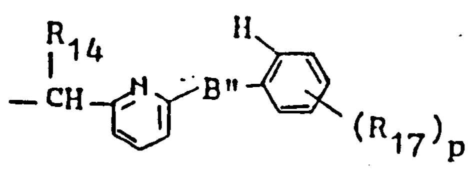

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et B" représente un atome d'oxygène ou un atome de soufre,

- soit un groupement :

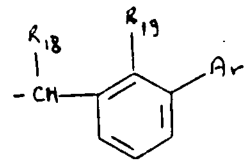

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,

- soit un groupement :

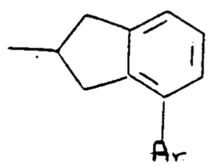

ou

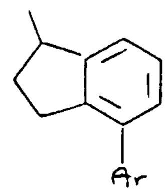

- soit un groupement :

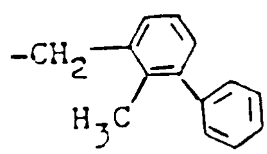

- soit un groupement :

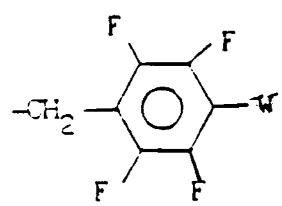

avec W = H, $CH_3$, $OCH_3$

à la condition que $R_1$ ne représente pas un radical $CF_3$ si R = H alkyle caractérisé en ce que l'on soumet un composé de formule (II) :

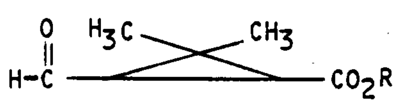

(II)

dans laquelle R conserve la même signification que précédemment à l'action d'un composé de formule V :

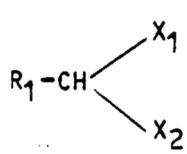

(V)

dans laquelle $X_1$, $X_2$ et $R_1$ conservent la même signification que précédemment en présence d'une base pour obtenir le composé de formule IV :

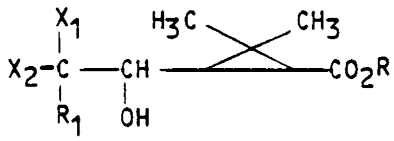

(IV)

que l'on soumet à l'action d'un agent de sulfonylation ou de phosphorylation ou de thiophosphorylation ou d'acylation pour obtenir le composé de formule I correspondant, que l'on transforme éventuellement en acide correspondant que l'on soumet éventuellement à un agent d'estérification pour obtenir un nouveau composé de formule I.

**2.** Procédé selon la revendication 1, caractérisé en ce que l'on utilise au départ un compose de formule (V) dans laquelle $X_1$ et $X_2$ représentent un atome de chlore, de brome ou d'iode.

**3.** Procédé selon la revendication 2, caractérisé en ce que l'on utilise au départ un composé de formule (V) dans laquelle $X_1$ et $X_2$ représentent un atome de brome.

**4.** Procédé selon l'une quelconque des revendications 1 à 3, caractérisé en ce que l'on utilise au départ un composé de formule (V) dans laquelle $R_1$ représente un atome de brome, de chlore ou de fluor.

**5.** Procédé selon l'une quelconque des revendications 1 à 3, caractérisé en ce que l'on utilise au départ un composé de formule (V) dans laquelle $R_1$ représente un radical trifluorométhyle.

**6.** Procédé selon l'une quelconque des revendications 1 à 5, caractérisé en ce que l'on utilise au départ un composé de formule (II) dans laquelle R représente :

- soit un atome d'hydrogène,
- soit un radical alkyle linéaire ou ramifié éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

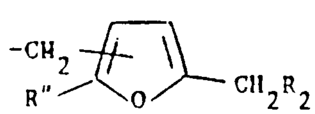

dans lequel le substituant R" représente un atome d'hydrogène ou un radical méthyle et le substituant $R_2$ un aryle monocyclique ou un groupement $-CH_2{\equiv}CH$ et notamment un groupement 5-benzyl 3-furyl méthyle,

- soit un groupement :

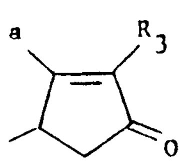

dans lequel a représente un atome d'hydrogène ou un radical méthyle et $R_3$ représente un radical organique aliphatique comportant de 2 à 6 atomes de carbone et une ou plusieurs

insaturations carbone-carbone et notamment te radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- soit un groupement :

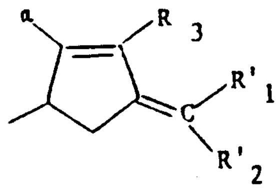

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

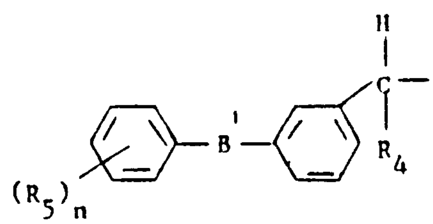

dans lequel B' représente un atome d'oxygène ou de soufre, un groupement

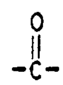

ou $-CH_2$ ou un groupement sulfoxyde ou un groupement sulfone et $R_4$ représente un atome d'hydrogène, un radical $-C\equiv N$, un radical méthyle, un radical $-CONH_2$, un radical $-CSNH_2$ ou un radical $-C\equiv CH$, $R_5$ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,

- soit un groupement :

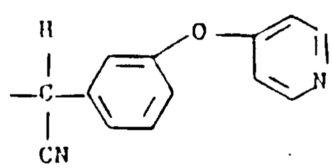

- soit un groupement :

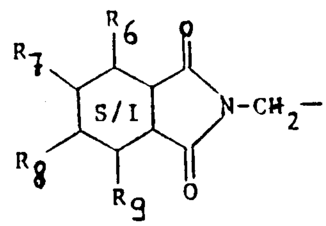

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

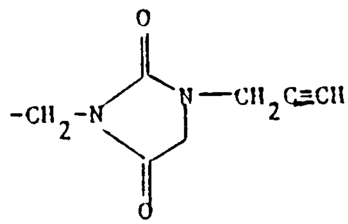

- soit un groupement :

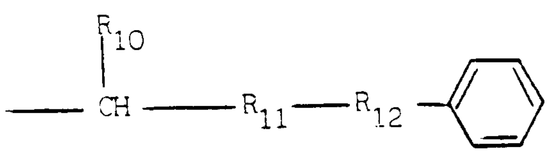

dans lequel $R_{10}$ représente un atome d'hydrogène ou un radical CN, $R_{12}$ représente un radical $-CH_2$-ou un atome d'oxygène, $R_{11}$ représente un radical thiazolyle ou thiadiazolyle dont la liaison avec

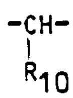

peut se trouver à l'une quelconque des positions disponibles, $R_{12}$ étant lié à $R_{11}$ par l'atome de carbone compris entre l'atome de soufre et un atome d'azote,

- soit un groupement :

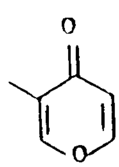

- soit un groupement :

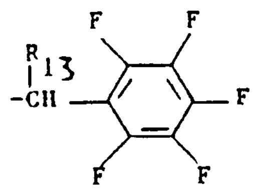

dans lequel $R_{13}$ représente un atome d'hydrogène ou un radical CN,

- soit un groupement :

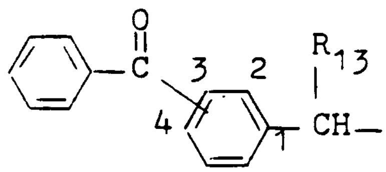

dans lequel $R_{13}$ est défini comme ci-dessus, et le radical benzoyle est en position 3 ou 4,

- soit un groupement :

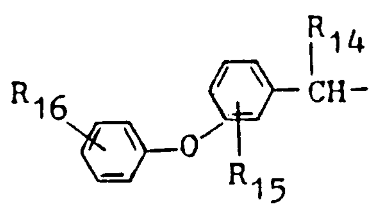

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupement :

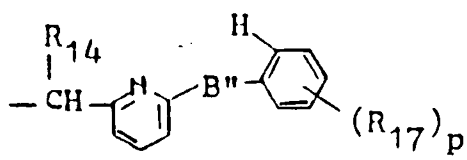

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et B" représente un atome d'oxygène ou un atome de soufre,

- soit un groupement :

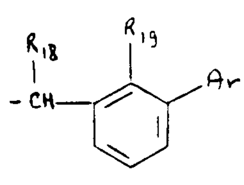

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,

- soit un groupement :

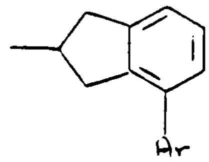

ou

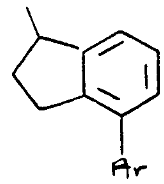

**7.** Procédé selon la revendication 6, caractérisé en ce que l'on utilise au départ un composé de formule (II) dans laquelle R représente un radical :

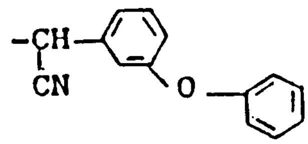

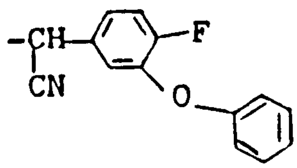

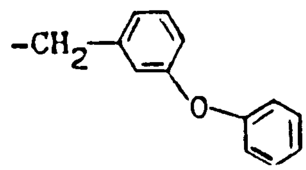

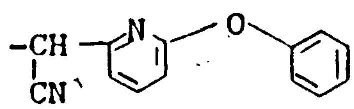

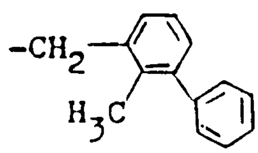

ou 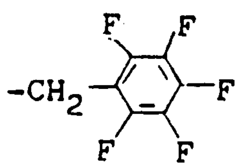 ou 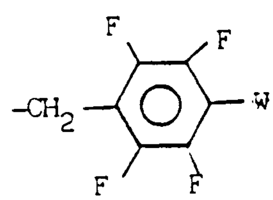

avec W =H, $CH_3$, $OCH_3$

ou 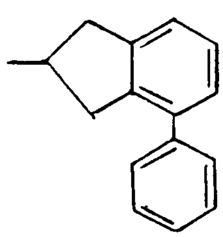

**8.** Procédé selon la revendication 1, caractérisé en ce que l'on soumet le composé de formule (IV) à l'action d'un agent de sulfonylation choisi de manière telle que l'on prépare les composés de formule (I) dans laquelle Y représente un radical $SO_2alc_1$, $alc_1$ représentant un radical alkyle ou alkényle renfermant jusqu'à 8 atomes de carbone.

**9.** Procédé selon la revendication 8, caractérisé en ce que l'on soumet le composé de formule (IV) à l'action d'un agent de sulfonylation choisi de manière telle que l'on prépare les composés de formule

(I) dans laquelle Y représente un radical $SO_2CH_3$.

**10.** Procédé selon la revendication 1, caractérisé en ce que l'on soumet le composé de formule (IV) à l'action d'un agent de sulfonylation choisi de manière telle que l'on prépare les composés de formule (I) dans laquelle Y représente un radical $SO_2Ar$, Ar conservant la même signification que précédemment.

**11.** Application des composés de formule I tels que définis à l'une quelconque des revendications 1 à 10 caractérisées en ce que l'on soumet un composé de formule I à l'action d'un agent de réduction pour obtenir le composé de formule III correspondant :

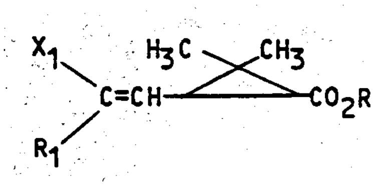

(III)

dans laquelle $X_1$, $R_1$ et R conservent leur signification précédente.

**12.** Application selon la revendication 12, caractérisée en ce que le composé de formule I répond à la formule I' :

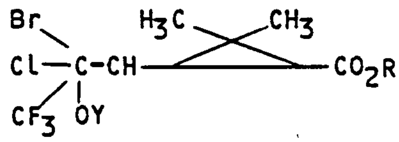

(I')

dans laquelle Y et R conservent la même signification que précédemment et le composé de formule III à la formule III' :

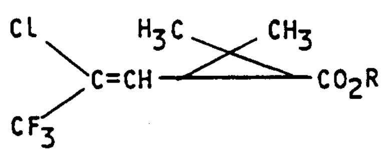

(III')

**13.** Application selon la revendication 12, caractérisée en ce que le composé de formule I répond à la formule I" :

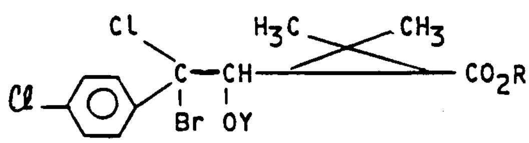

(I")

dans laquelle Y et R conservent la même signification que précédemment et le composé III obtenu à la formule III" :

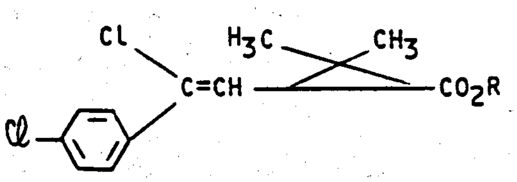

(III")

## Claims

## Claims for the following Contracting States : AT, BE, CH, DE, FR, GB, GR, IT, LI, LU, NL

**1.** The compounds of formula (I):

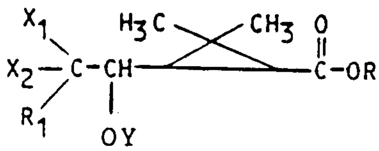

(I)

in which $X_1$ and $X_2$, identical to or different from each other, represent a halogen atom, $R_1$ represents a halogen atom, an alkyl radical containing up to 8 carbon atoms, an optionally substituted aryl radical containing up to 14 carbon atoms, a perfluoroalkyl radical containing up to 8 carbon atoms, a -C≡N radical, a

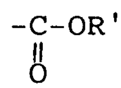

radical in which R' represents an alkyl radical containing up to 8 carbon atoms, Y represents a radical chosen from the group constituted by:

- the $SO_2alk_1$ radicals, $alk_1$ representing a saturated or unsaturated, linear or branched alkyl radical optionally substituted by one or more functional groups;
- the $SO_2A_r$ radicals, $A_r$ representing an aryl radical containing up to 14 carbon atoms, optionally substituted by one or more functional groups;
- the

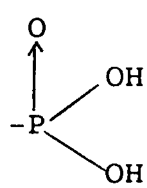

or

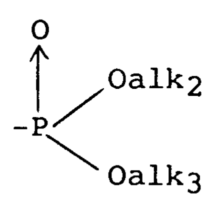

radicals $alk_2$ and $alk_3$, identical to or different from each other, representing a saturated or unsaturated, linear or branched alkyl radical containing up to 8 carbon atoms, optionally substituted by one or more functional groups or $alk_2$ and $alk_3$ being able to form with the

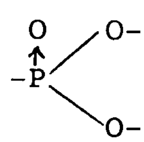

radical,

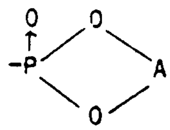

a ring, A representing a saturated or unsaturated, linear or branched carbonated chain, optionally substituted by one or more functional groups and containing up to 6 carbon atoms;
- the

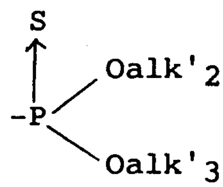

radicals, alk'$_2$ and alk'$_3$ representing the same values as alk$_2$ and alk$_3$;
- the

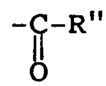

radical, R" representing an aryl radical and R represents
- either a hydrogen atom,
- or an optionally substituted linear or branched alkyl radical containing 1 to 8 carbon atoms,
- or a benzyl radical optionally substituted by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy radical and halogen atoms,
- or a group:

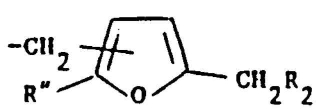

in which the substituent R" represents a hydrogen atom or a methyl radical and the substituent $R_2$ represents a monocyclic aryl radical or a -C≡CH group and in particular a 5-benzyl-3-furyl methyl group,
- or a group:

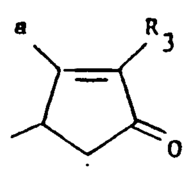

in which a represents a hydrogen atom or a methyl radical and $R_3$ represents an aliphatic organic radical containing 2 to 6 carbon atoms and one or more carbon-carbon unsaturations and in particular one of the following radicals: $-CH_2-CH=CH_2$, $-CH_2-CH=CH-CH_3$, $-CH_2-CH=CH-CH=CH_2$, $-CH_2-CH=CH-CH_2-CH_3$, $-CH_2-C \equiv CH$,
- or a group:

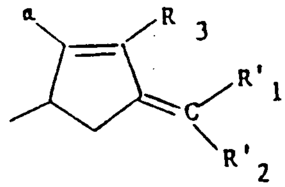

in which a represents a hydrogen atom or a methyl radical, $R_3$ keeps the same meaning as previously, $R'_1$ and $R'_2$, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,

- or a group:

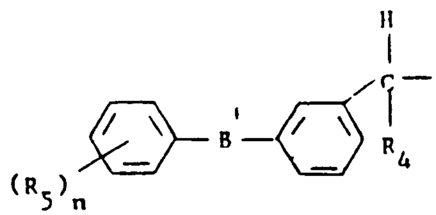

in which B' represents an oxygen or sulphur atom, a

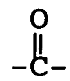

or $-CH_2$ group or a sulphoxide group or a sulphone group and $R_4$ represents a hydrogen atom, a C≡N radical, a methyl radical, a $-CONH_2$ radical, a $-CSNH_2$ radical or a -C≡CH radical, $R_5$ represents a halogen atom or a methyl radical and n represents a number equal to 0, 1 or 2, and notably one of the following groups: 3-phenoxy benzyl, alpha-cyano 3-phenoxy benzyl, alpha-ethynyl 3-phenoxy benzyl, 3-benzoyl benzyl, 1-(3-phenoxy phenyl) ethyl or alpha thioamido 3-phenoxy benzyl,

- or a group:

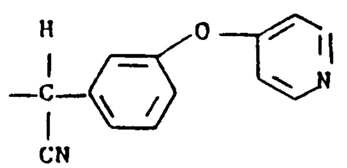

- or a group:

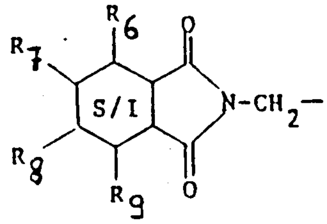

in which the substituents $R_6$, $R_7$, $R_8$, $R_9$ represent a hydrogen atom, a chlorine atom, or a methyl radical and in which S/I symbolizes an aromatic ring or a similar dihydro or tetrahydro

ring,

- or a group:

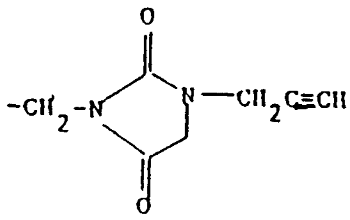

- or a group:

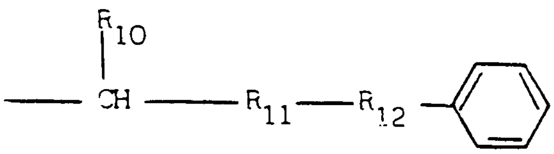

in which $R_{10}$ represents a hydrogen atom or a CN radical, $R_{12}$ represents a $-CH_2$ radical or an oxygen atom, $R_{11}$ represents a thiazolyl or thiadiazolyl radical of which the bond with

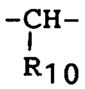

can be found in any of the available positions, $R_{12}$ being linked to $R_{11}$ by the carbon atom contained between the sulphur atom and a nitrogen atom,

- or a group:

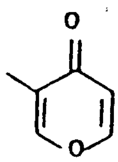

- or a group

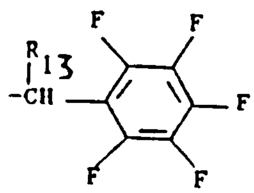

in which $R_{13}$ represents a hydrogen atom or a CN radical,

- or a group:

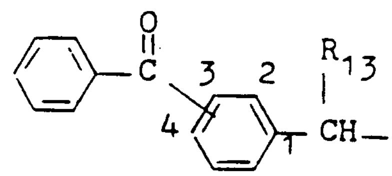

in which $R_{13}$ is defined as above, and the benzoyl radical is in position 3 or 4,

- or a group:

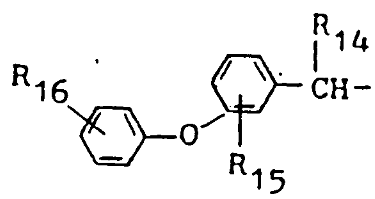

in which $R_{14}$ represents a hydrogen atom, a methyl, ethynyl or cyano radical and $R_{15}$ and $R_{16}$, being different, represent a hydrogen, fluorine or bromine atom,

- or a group:

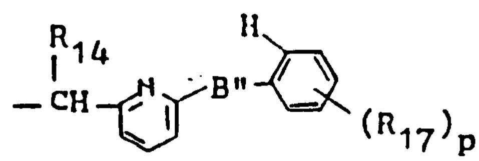

in which $R_{14}$ is defined as above, each of the $R_{17}$ substituents represents independently one of the following groups: alkyl containing 1 to 4 carbon atoms, alkoxy containing 1 to 4 carbon atoms, alkylthio containing 1 to 4 carbon atoms, alkylsulphonyl containing 1 to 4 carbon atoms, trifluoromethyl, 3,4-methylenedioxy, chloro, fluoro or bromine, p represents a number equal to 0, 1 or 2 and B" represents an oxygen atom or a sulphur atom,

- or a group:

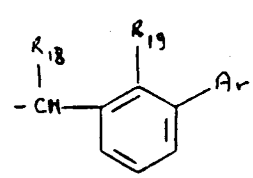

in which $R_{18}$ represents a hydrogen atom, a methyl, ethynyl or cyano radical and $R_{19}$, being different, represents a hydrogen, fluorine or bromine atom and Ar represents an aryl radical containing up to 14 carbon atoms,

- or a group:

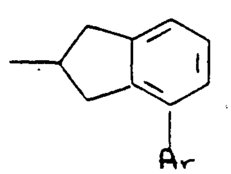

or

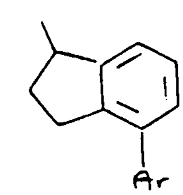

- or a group:

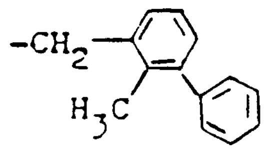

- or a group:

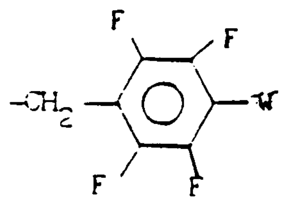

with W=H, $CH_3$, $OCH_3$

on condition that $R_1$ does not represent a $CF_3$ radical if R = H alkyl.

**2.** The compounds of formula (I) as defined in claim 1 in which R represents a radical:

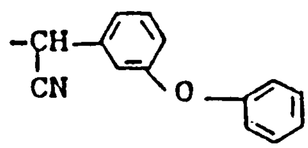

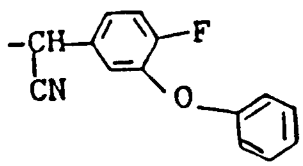

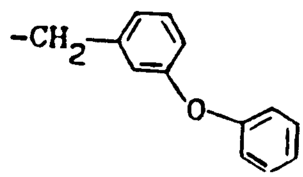

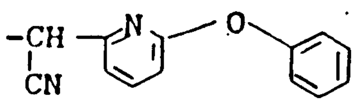

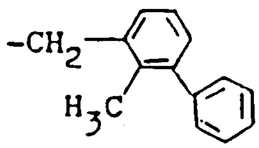

or

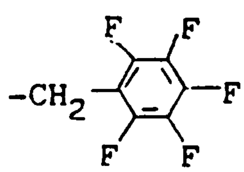

or

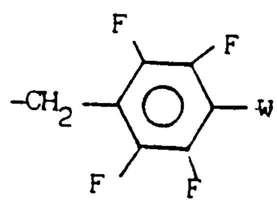

with W=H, $CH_3$, $OCH_3$

or

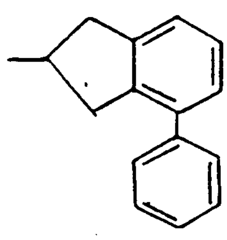

**3.** The compounds of formula (I) as defined in claim 1 or 2 in which $X_1$ and $X_2$ represent a chlorine, bromine or iodine atom.

**4.** The compounds of formula (I) as defined in claim 3 in which $X_1$ and $X_2$ represent a bromine atom.

**5.** The compounds of formula (I) as defined in any one of claims 1 to 4 in which $R_1$ represents a bromine, chlorine or fluorine atom.

**6.** The compounds of formula (I) as defined in any one of claims 1 to 5 in which Y represents an $SO_2alk_1$ radical, $alk_1$ representing an alkyl or alkenyl radical containing up to 8 carbon atoms.

**7.** The compounds of formula (I) as defined in claim 6 in which Y represents an $SO_2CH_3$ radical.

**8.** The compounds of formula (I) as defined in any one of claims 1 to 5 in which Y represents an $SO_2Ar$ radical, Ar keeping the same meaning as previously.

**9.** The compounds of formula (I) as defined in claim 1 of which the names follow:

- cyano (3-phenoxy-phenyl) methyl 1R-[1-alpha-(S)-3-alpha-(R)]-2,2-dimethyl-3-[1-(methylsulphonyloxy)-2,2,2-tribromoethyl]-cyclopropane carboxylate,
- cyano [(3-phenoxy-4-fluoro)-phenyl] methyl 1R-[1-alpha-(S)-3-alpha-(R)]-2,2-dimethyl-3-[2,2,2-tribromo-1-methyl-sulphonyloxy ethyl]-cyclopropane carboxylate,
- cyano (3-phenoxyphenyl) methyl 1R-[1-alpha, 3-alpha (RS*,RS*)]-2,2-dimethyl-3-[2-trifluoromethyl-2-bromo-2-chloro-1-methyl-sulphonyloxy-ethyl]-cyclopropane carboxylate.

**10.** Preparation process for compounds of formula (I) of claim 1, in which $X_1$, $X_2$ and R keep the same meaning as in claim 1, $R_1$ keeps the same meaning as in claim 1 and can also represent a perfluoroalkyl radical containing up to 8 carbon atoms, Y keeps the same meaning as in claim 1 and can also represent a

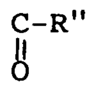

radical in which R" represents an optionally substituted alkyl radical, characterized in that a compound of formula:

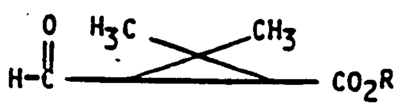

(II)

in which R keeps the same meaning as previously, is subjected to the action of a compound of formula (V):

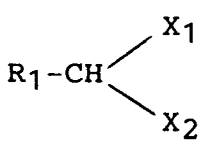

(V)

in which $X_1$, $X_2$ and $R_1$ keep the same meaning as previously, in the presence of a base, in order to obtain the compound of formula (IV):

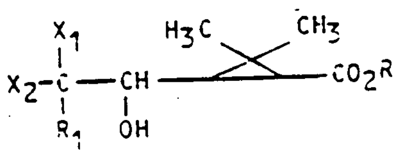

(IV)

which is subjected to the action of a sulphonylation or phosphonylation agent or a thiophosphorylation or acylation agent, in order to obtain the corresponding compound of formula (I):

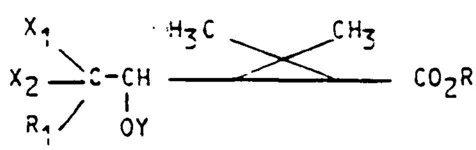

which is optionally converted into a corresponding acid which is optionally subjected to an esterification agent in order to obtain a new compound of formula (I).

**Claims for the following Contracting State : ES**

**1.** Preparation process for compounds of formula (I):

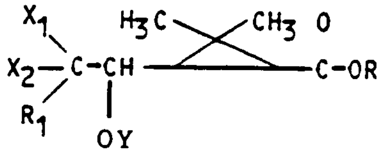

(I)

in which $X_1$ and $X_2$, identical to or different from each other, represent a halogen atom, $R_1$ represents a halogen atom, an alkyl radical containing up to 8 carbon atoms, an optionally substituted aryl radical containing up to 14 carbon atoms, a perfluoroalkyl radical containing up to 8 carbon atoms, a -C≡N radical, a

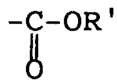

radical in which R' represents an alkyl radical containing up to 8 carbon atoms, Y represents a radical chosen from the group constituted by:

- the $SO_2alk_1$ radicals, $alk_1$ representing a saturated or unsaturated, linear or branched alkyl radical optionally substituted by one or more functional groups;
- the $SO_2A_r$ radicals, $A_r$ representing an aryl radical containing up to 14 carbon atoms, optionally substituted by one or more functional groups;
- the

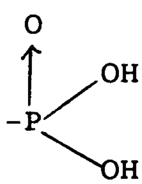

or

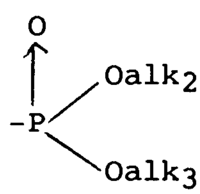

radicals $alk_2$ and $alk_3$, identical to or different from each other, representing a saturated or unsaturated, linear or branched alkyl radical containing up to 8 carbon atoms, optionally substituted by one or more functional groups or $alk_2$ and $alk_3$ being able to form with the

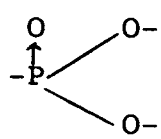

radical, a

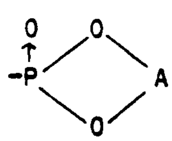

ring, A representing a saturated or unsaturated, linear or branched carbonated chain, optionally substituted by one or more functional groups and containing up to 6 carbon atoms;
- the

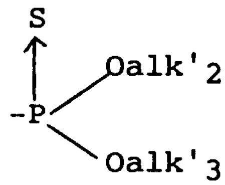

radicals, alk'$_2$ and alk'$_3$ representing the same values as alk$_2$ and alk$_3$;

- the

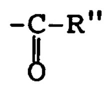

radical, R" representing an aryl radical and R represents

- either a hydrogen atom,
- or an optionally substituted linear or branched alkyl radical containing 1 to 8 carbon atoms,
- or a benzyl radical optionally substituted by one or more radicals chosen from the group constituted by alkyl radicals containing 1 to 4 carbon atoms, the alkenyl radicals containing 2 to 6 carbon atoms, the alkenyloxy radicals containing 2 to 6 carbon atoms, the alkadienyl radicals containing 4 to 8 carbon atoms, the methylenedioxy radical and halogen atoms,
- or a group:

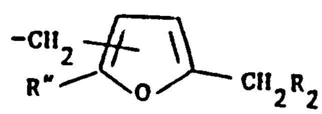

  in which the R" substituent represents a hydrogen atom or a methyl radical and the R$_2$ substituent represents a monocyclic aryl radical or a -C≡CH group, notably a 5-benzyl-3-furyl methyl group,
- or a group:

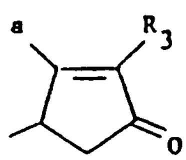

  in which a represents a hydrogen atom or a methyl radical and R$_3$ represents an aliphatic organic radical containing 2 to 6 carbon atoms and one or more carbon-carbon unsaturations and in particular the following radicals: $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,
- or a group:

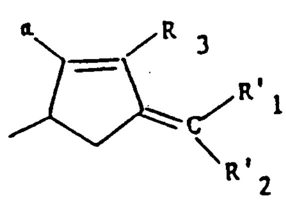

in which a represents a hydrogen atom or a methyl radical, $R_3$ keeps the same meaning as previously, $R'_1$ and $R'_2$, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,

- or a group:

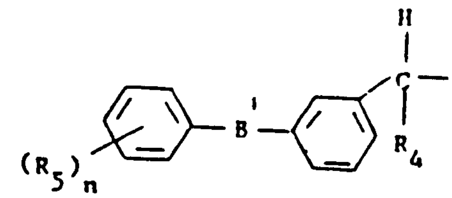

in which B' represents an oxygen or sulphur atom, a

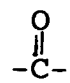

or $-CH_2$ group or a sulphoxide group or a sulphone group and $R_4$ represents a hydrogen atom, a -C≡N radical, a methyl radical, a $-CONH_2$ radical, a $-CSNH_2$ radical or a -C≡CH radical, $R_5$ represents a halogen atom or a methyl radical and n represents a number equal to 0, 1 or 2, and in particular one of the following groups: 3-phenoxy benzyl, alpha-cyano 3-phenoxy benzyl, alpha-ethynyl 3-phenoxy benzyl, 3-benzoyl benzyl, 1-(3-phenoxy phenyl)-ethyl or alpha-thioamido-3-phenoxy benzyl,

- or a group:

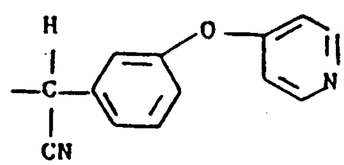

- or a group:

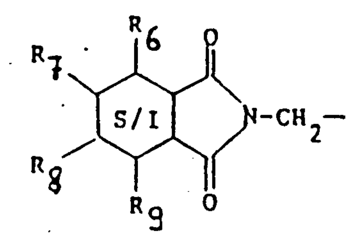

in which the substituents $R_6$, $R_7$, $R_8$, $R_9$ represent a hydrogen atom, a chlorine atom, or a methyl radical, and in which S/I symbolizes an aromatic ring or a similar dihydro or tetrahydro ring,

- or a group:

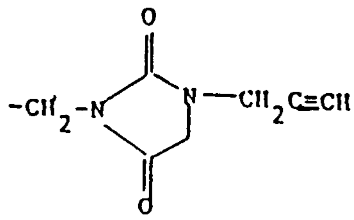

- or a group:

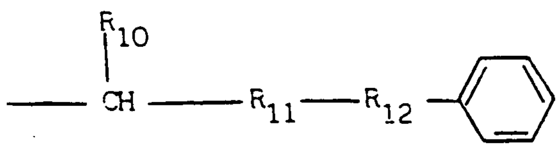

in which $R_{10}$ represents a hydrogen atom or a CN radical, $R_{12}$ represents a $-CH_2$ radical or an oxygen atom, $R_{11}$ represents a thiazolyl or thiadiazolyl radical whose bond with

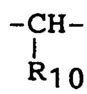

can be found in any of the available positions, $R_{12}$ being linked to $R_{11}$ by the carbon atom contained between the sulphur atom and a nitrogen atom,

- or a group:

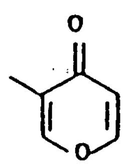

- or a group:

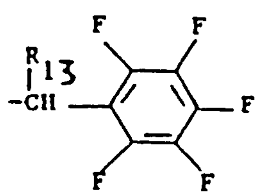

in which $R_{13}$ represents a hydrogen atom or a CN radical,

- or a group:

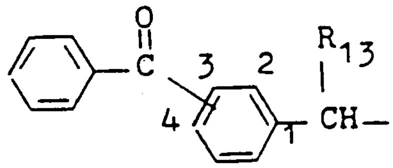

in which $R_{13}$ is defined as above, and the benzoyl radical is in position 3 or 4,

- or a group:

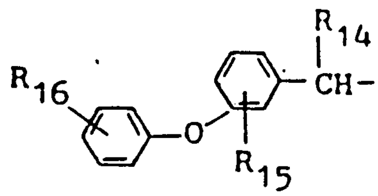

in which $R_{14}$ represents a hydrogen atom, a methyl, ethynyl or cyano radical and $R_{15}$ and $R_{16}$, being different, represent a hydrogen, fluorine or bromine atom,

- or a group:

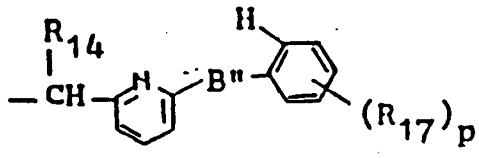

in which $R_{14}$ is defined as above, each of the $R_{17}$ substituents represents independently one of the following groups: alkyl containing 1 to 4 carbon atoms, alkoxy containing 1 to 4 carbon atoms, alkylthio containing 1 to 4 carbon atoms, alkylsulphonyl containing 1 to 4 carbon atoms, trifluoromethyl, 3,4-methylene dioxy, chloro, fluoro or bromine, p represents a number equal to 0, 1 or 2 and B" represents an oxygen atom or a sulphur atom,

- or a group:

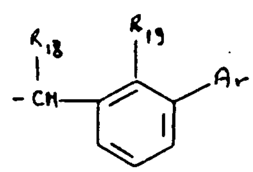

in which $R_{18}$ represents a hydrogen atom, a methyl, ethynyl or cyano radical and $R_{19}$, being different, represents a hydrogen, fluorine or bromine atom and Ar represents an aryl radical containing up to 14 carbon atoms,

- or a group:

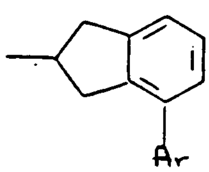

or

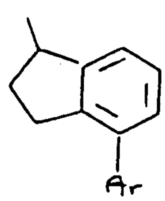

- or a group:

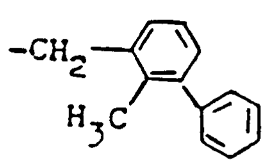

- or a group:

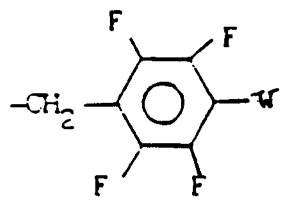

with W=H, $CH_3$, $OCH_3$

on condition that $R_1$ does not represent a $CF_3$ radical is R = H alkyl, characterized in that a compound of formula (II):

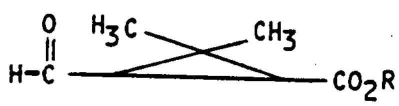

(II)

in which R keeps the same meaning as previously, is subjected to the action of a compound of formula (V):

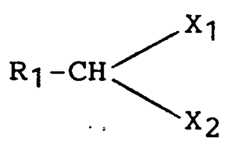

(V)

in which $X_1$, $X_2$ and $R_1$ keep the same meaning as previously, in the presence of a base, in order to obtain the compound of formula (IV):

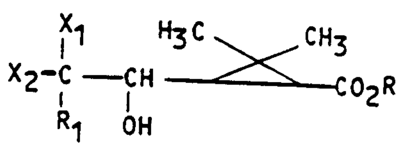

(IV)

which is subjected to the action of a sulphonylation or phosphorylation agent or a thiophosphorylation or acylation agent, in order to obtain the corresponding compound of formula (I), which is optionally converted into a corresponding acid, which is optionally subjected to an esterification agent in order to obtain a new compound of formula (I).

2. Process according to claim 1, characterized in that a compound of formula (V) in which $X_1$ and $X_2$ represent a chlorine, bromine or iodine atom is used at the start.

3. Process according to claim 2, characterized in that a compound of formula (V) in which $X_1$ and $X_2$ represent a bromine atom is used at the start.

4. Process according to any one of claims 1 to 3, characterized in that a compound of formula (V) in which $R_1$ represents a bromine, chlorine or fluorine atom is used at the start.

5. Process according to any one of claims 1 to 3, characterized in that a compound of formula (V) in which $R_1$ represents a trifluoromethyl radical is used at the start.

6. Process according to any one of claims 1 to 5, characterized in that a compound of formula (II) is used

at the start in which R represents:

- either a hydrogen atom,
- or an optionally substituted linear or branched alkyl radical containing 1 to 8 carbon atoms,
- or a benzyl radical optionally substituted by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy radical and halogen atoms,
- or a group:

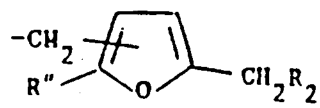

in which the R" substituent represents a hydrogen atom or a methyl radical and the $R_2$ substituent represents a monocyclic aryl radical or a $-CH_2{\equiv}CH$ group and in particular a 5-benzyl-3-furyl methyl group,
- or a group:

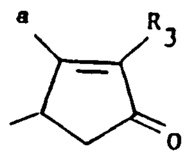

in which a represents a hydrogen atom or a methyl radical and $R_3$ represents an aliphatic organic radical containing 2 to 6 carbon atoms and one or more carbon-carbon unsaturations and in particular one of the following radicals: $-CH_2-CH=CH_2$,$-CH_2-CH=CH-CH_3$, $-CH_2-CH=CH=CH=CH_2$, $-CH_2-CH=CH-CH_2-CH_3$, $-CH_2-C{\equiv}CH$,
- or a group:

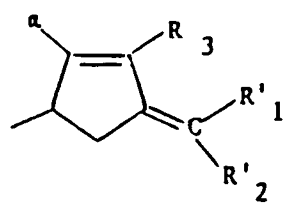

in which a represents a hydrogen atom or a methyl radical, $R_3$ keeps the same meaning as previously, $R'_1$ and $R'_2$, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,
- or a group:

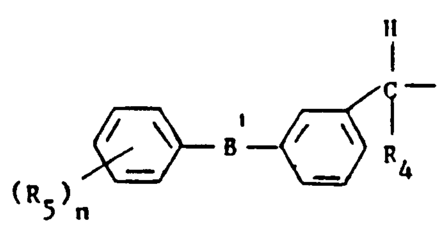

in which B' represents an oxygen or sulphur atom, a

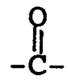

or $-CH_2$ group or a sulphoxide group or a sulphone group and $R_4$ represents a hydrogen atom, a $-C{\equiv}N$ radical, a methyl radical, a $-CONH_2$ radical, a $-CSNH_2$ radical or a $-C{\equiv}CH$ radical, $R_5$ represents a halogen atom or a methyl radical and n represents a number equal to 0, 1 or 2, and in particular one of the following groups: 3-phenoxy benzyl, alpha-cyano 3-phenoxy benzyl, alpha-ethynyl 3-phenoxy benzyl, 3-benzoyl benzyl, 1-(3-phenoxy phenyl) ethyl or alpha-thioamido 3-phenoxy benzyl,

- or a group:

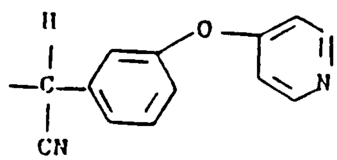

- or a group:

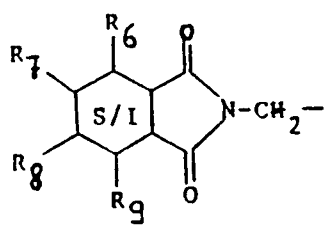

in which the substituents $R_6$, $R_7$, $R_8$, $R_9$ represent a hydrogen atom, a chlorine atom, or a methyl radical and in which S/I symbolizes an aromatic ring or a similar dihydro or tetrahydro ring,

- or a group:

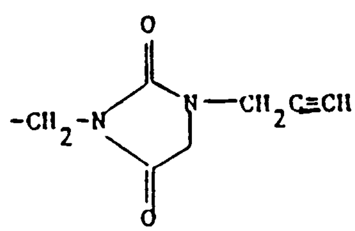

- or a group:

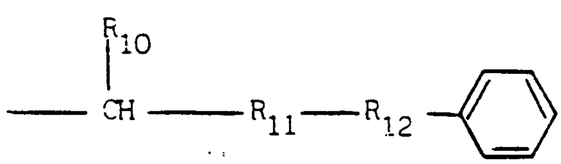

in which $R_{10}$ represents a hydrogen atom or a CN radical, $R_{12}$ represents a $-CH_2$ radical or an oxygen atom, $R_{11}$ represents a thiazolyl or thiadiazolyl radical whose bond with

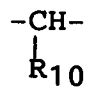

can be found in any of the available positions, $R_{12}$ being linked to $R_{11}$ by the carbon atom contained between the sulphur atom and a nitrogen atom,

- or a group:

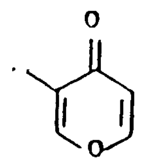

- or a group:

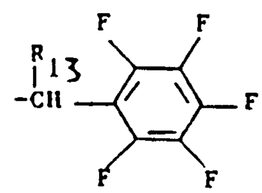

in which $R_{13}$ represents a hydrogen atom or a CN radical,

- or a group:

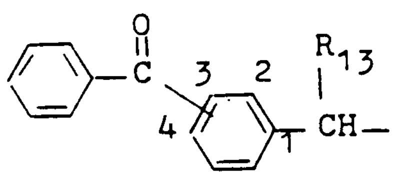

in which $R_{13}$ is defined as above, and the benzoyl radical is in position 3 or 4,

- or a group:

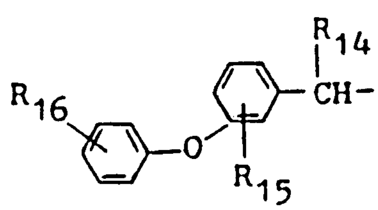

in which $R_{14}$ represents a hydrogen atom, a methyl, ethynyl or cyano radical and $R_{15}$ and $R_{16}$, being different, represent a hydrogen, fluorine or bromine atom,

- or a group:

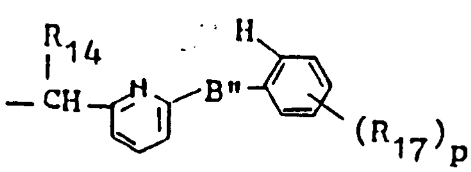

in which $R_{14}$ is defined as above, each of the $R_{17}$ substituents represents independently one of the following groups: alkyl containing 1 to 4 carbon atoms, alkoxy containing 1 to 4 carbon atoms, alkylthio containing 1 to 4 carbon atoms, alkylsulphonyl containing 1 to 4 carbon atoms, trifluoromethyl, 3,4-methylene dioxy, chloro, fluoro or bromine, p represents a number equal to 0, 1 or 2 and B" represents an oxygen atom or a sulphur atom,

- or a group:

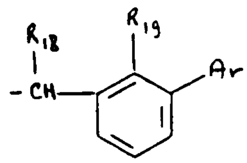

in which $R_{18}$ represents a hydrogen atom, a methyl, ethynyl or cyano radical and $R_{19}$, being different, represents a hydrogen, fluorine or bromine atom and Ar represents an aryl radical containing up to 14 carbon atoms,

- or a group:

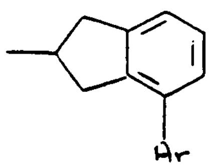

or

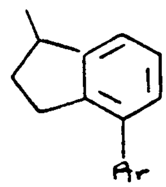

**7.** Process according to claim 6, characterized in that a compound of formula (II) is used at the start in which R represents a radical:

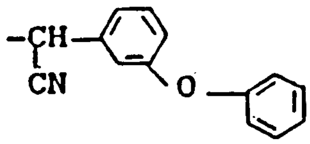

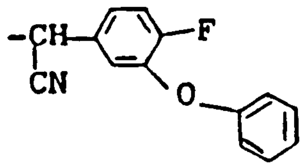

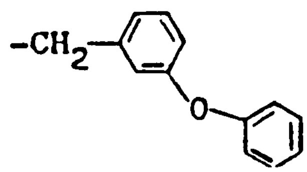

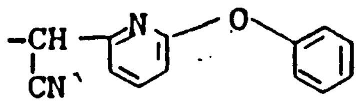

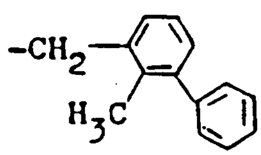

or 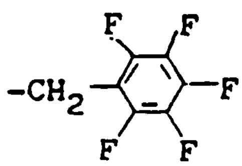 or 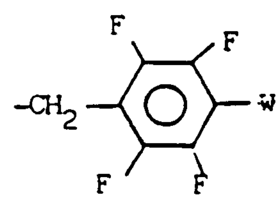

with W=H, $CH_3$, $OCH_3$

or 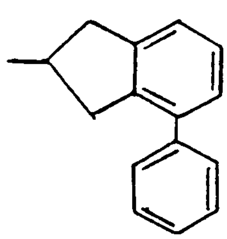

8. Process according to claim 1, characterized in that the compound of formula (IV) is subjected to the action of a sulphonylation agent chosen in such a way that the compounds of formula (I) in which Y represents an $SO_2alk_1$ radical, $alk_1$ representing an alkyl or alkenyl radical containing up to 8 carbon atoms are prepared.

9. Process according to claim 8, characterized in that the compound of formula (IV) is subjected to the action of a sulphonylation agent chosen in such a way that the compounds of formula (I) in which Y

represents an $SO_2CH_3$ radical are prepared.

**10.** Process according to claim 1, characterized in that the compound of formula (IV) is subjected to the action of a sulphonylation agent chosen in such a way that the compounds of formula (I) in which Y represents an $SO_2Ar$ radical, Ar keeping the same meaning as previously, are prepared.

**11.** Use of the compounds of formula (I) as defined in any one of claims 1 to 10, characterized in that a compound of formula (I) is subjected to the action of a reduction agent in order to obtain the corresponding compound of formula (III):

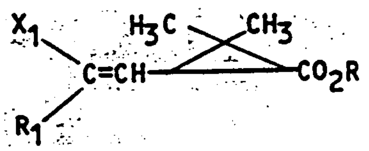

(III)

in which $X_1$, $R_1$ and R keep their previous meaning.

**12.** Use according to claim 11, characterized in that the compound of formula (I) corresponds to formula (I'):

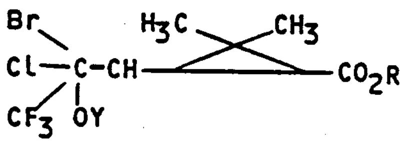

(I')

in which Y and R keep the same meaning as previously and the compound of formula (III) corresponds to formula (III'):

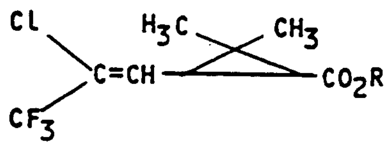

(III')

**13.** Use according to claim 12, characterized in that the compound of formula (I) corresponds to formula (I"):

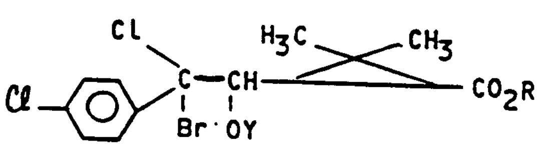

(I")

in which Y and R keep the same meaning as previously and the compound (III) obtained corresponds to formula (III"):

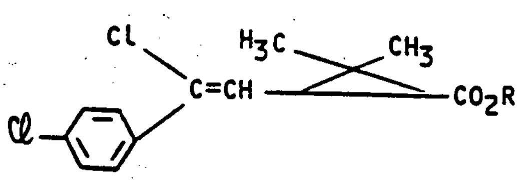

(III")

## Patentansprüche

**Patentansprüche für folgende Vertragsstaaten : AT, BE, CH, DE, FR, GB, GR, IT, LI, LU, NL**

**1.** Verbindungen der Formel (I)

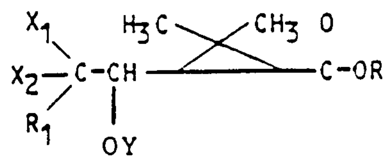

(I)

worin $X_1$ und $X_2$, die identisch oder voneinander verschieden sind, für ein Halogenatom stehen, $R_1$ ein Halogenatom, einen Alkylrest mit bis zu 8 Kohlenstoffatomen, einen gegebenenfalls substituierten Arylrest mit bis zu 14 Kohlenstoffatomen, einen Perfluoralkylrest mit bis zu 8 Kohlenstoffatomen, einen Rest -C≡N, einen Rest

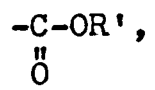

worin R' fur einen Alkylrest mit bis zu 8 Kohlenstoffatomen steht, bedeutet, Y für einen Rest steht, ausgewählt unter

- den Resten $SO_2alc_1$, wobei $alc_1$ einen gesättigten oder ungesättigten, linearen oder verzweigten Alkylrest bedeutet, der gegebenenfalls durch eine oder mehrere funktionelle Gruppen substituiert ist;
- den Resten $SO_2A_r$, wobei $A_r$ für einen Arylrest mit bis zu 14 Kohlenstoffatomen steht, der gegebenenfalls durch eine oder mehrere funktionelle Gruppen substituiert ist;
- den Resten

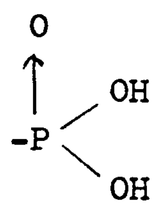

oder

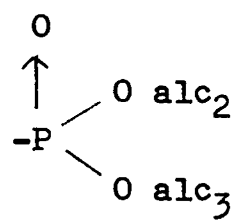

,

wobei $alc_2$ und $alc_3$ identisch oder voneinander verschieden sind und einen gesättigten oder ungesättigten, linearen oder verzweigten Alkylrest mit bis zu 8 Kohlenstoffatomen wiedergeben, der gegebenenfalls durch eine oder mehrere funktionelle Gruppen substituiert ist, oder $alc_2$ und $alc_3$ mit dem Rest

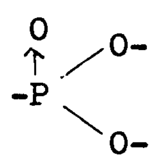

einen Ring

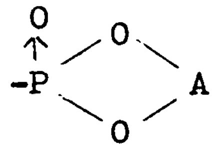

bilden können, wobei A eine gesättigte oder ungesättigte, lineare oder verzweigte Kohlenstoffkette, die gegebenenfalls durch eine oder mehrere funktionelle Gruppen substituiert ist und bis zu 6 Kohlenstoffatome aufweist, wiedergibt;

- den Resten

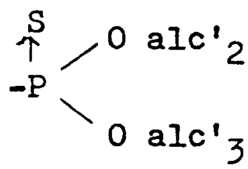

wobei alc'$_2$ und alc'$_3$ die Bedeutungen von alc$_2$ und alc$_3$ besitzen;

- dem Rest

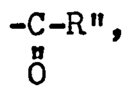

wobei R" einen Arylrest bedeutet; und R bedeutet:

- entweder ein Wasserstoffatom,
- oder einen gegebenenfalls substituierten, linearen oder verzweigten Alkylrest mit 1 bis 8 Kohlenstoffatomen,
- oder einen Benzylrest, der gegebenenfalls substituiert ist durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,
- oder eine Gruppe

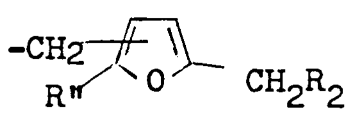

worin der Substituent R" ein Wasserstoffatom oder eine Methylgruppe bedeutet und der Substituent R$_2$ einen monocyclischen Arylrest oder eine Gruppe -C≡CH bedeutet, und insbesondere eine 5-Benzyl-3-furylmethylgruppe

- oder eine Gruppe

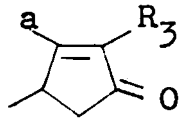

worin a ein Wasserstoffatom oder eine Methylgruppe bedeutet und R$_3$ einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2-CH=CH-CH_2-CH_3$, $-CH_2-C≡CH$ wiedergibt,

- oder eine Gruppe

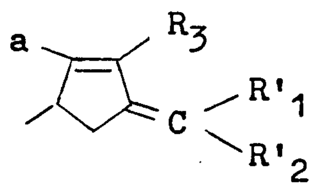

worin a für ein Wasserstoffatom oder eine Methylgruppe steht, $R_3$ die vorstehend angegebene Bedeutung besitzt, $R'_1$ und $R'_2$ identisch oder voneinander verschieden sind und ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

- oder eine Gruppe

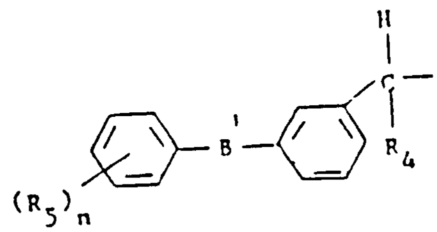

worin B' ein Sauerstoff- oder Schwefelatom, eine Gruppe

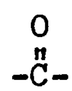

oder $-CH_2$ oder eine Sulfoxidgruppe oder eine Sulfongruppe wiedergibt und $R_4$ ein Wasserstoffatom, einen Rest -C≡N, eine Methylgruppe, einen Rest $-CONH_2$, einen Rest $-CSNH_2$ oder einen Rest -C≡CH bedeutet, $R_5$ für ein Halogenatom oder eine Methylgruppe steht und n eine ganze Zahl entsprechend 0, 1 oder 2 wiedergibt, und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzylgruppe,

- oder eine Gruppe

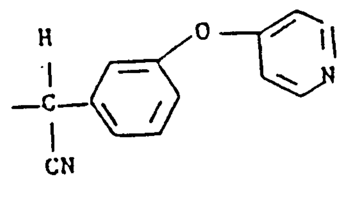

- oder eine Gruppe

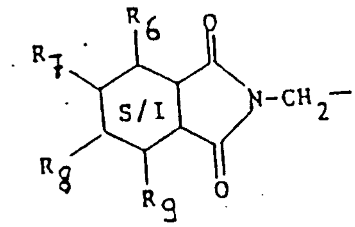

worin die Substituenten $R_6$, $R_7$, $R_8$, $R_9$ für ein Wasserstoffatom, ein Chloratom oder einen Methylrest stehen und worin S/I einen aromatischen Ring oder einen analogen Dihydro- oder Tetrahydroring symbolisiert,

- oder eine Gruppe

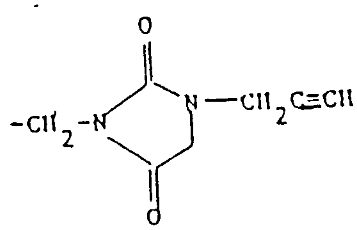

- oder eine Gruppe

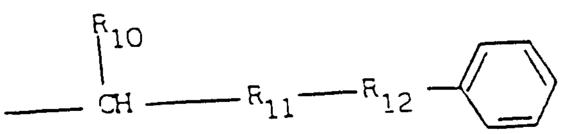

worin $R_{10}$ für ein Wasserstoffatom oder eine CN-Gruppe steht, $R_{12}$ einen Rest $-CH_2$ oder ein Sauerstoffatom bedeutet, $R_{11}$ eine Thiazolyl- oder Thiadiazolylgruppe wiedergibt, deren Bindung mit

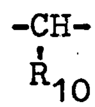

sich in irgendeiner der verfügbaren Positionen befinden kann, wobei $R_{12}$ an $R_{11}$ über ein Kohlenstoffatom zwischen dem Schwefelatom und einem Stickstoffatom gebunden ist,

- oder eine Gruppe

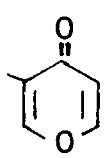

- oder eine Gruppe

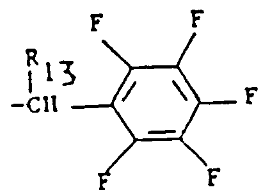

worin $R_{13}$ für ein Wasserstoffatom oder eine CN-Gruppe steht,

- oder eine Gruppe

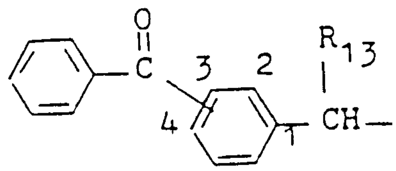

worin $R_{13}$ wie vorstehend definiert ist und der Benzoylrest sich in 3- oder 4-Stellung befindet,

- oder eine Gruppe

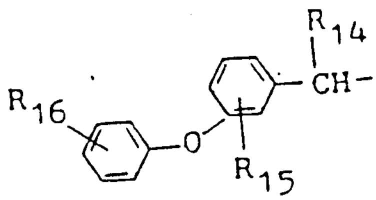

worin $R_{14}$ ein Wasserstoffatom, eine Methyl-, Ethinyl- oder Cyanogruppe bedeutet und $R_{15}$ und $R_{16}$, die voneinander verschieden sind, für ein Wasserstoff-, Fluor- oder Bromatom stehen,

- oder eine Gruppe

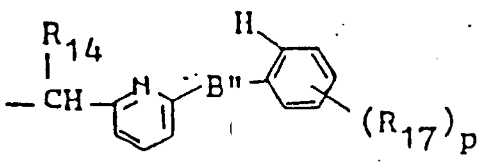

worin $R_{14}$ wie vorstehend definiert ist, jede der Gruppen $R_{17}$ unabhängig einen Alkylrest mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, 3,4-Methylendioxy, Chlor, Fluor oder Brom bedeutet, p für eine ganze Zahl entsprechend 0, 1 oder 2 steht und B" ein Sauerstoff- oder ein Schwefelatom wiedergibt,

- oder eine Gruppe

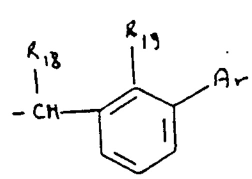

worin $R_{18}$ für ein Wasserstoffatom, eine Methyl-, Ethinyl- oder Cyanogruppe steht und $R_{19}$, welches verschieden ist, ein Wasserstoff-, Fluor- oder Bromatom bedeutet und Ar für einen Arylrest mit bis zu 14 Kohlenstoffatomen steht,

- oder eine Gruppe

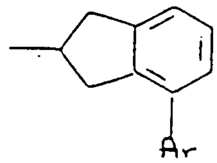

oder

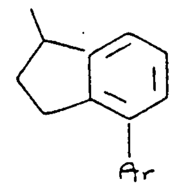

- oder eine Gruppe

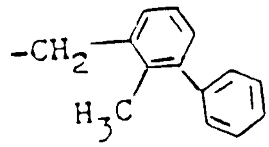

- oder eine Gruppe

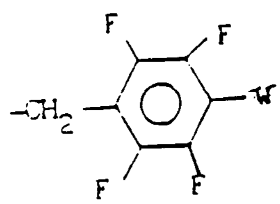

mit W = H, $CH_3$, $OCH_3$

unter der Bedingung, daß $R_1$ keine $CF_3$-Gruppe bedeutet, wenn R = H, Alkyl.

**2.** Verbindungen der Formel, wie in Anspruch 1 definiert, worin R einen der folgenden Reste bedeutet:

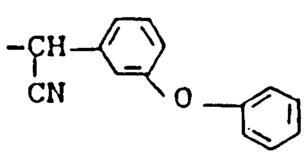

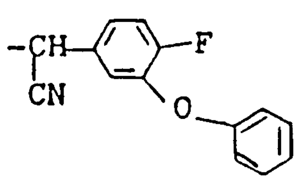

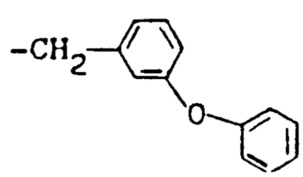

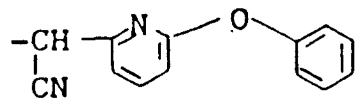

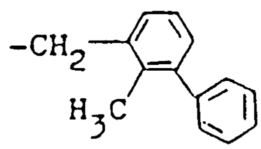

oder 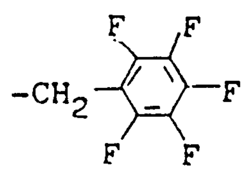 oder 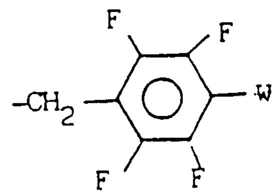

mit W=H, $CH_3$, $OCH_3$

oder 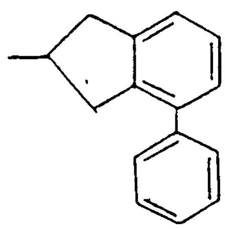

**3.** Verbindungen der Formel I, wie in Anspruch 1 oder 2 definiert, worin $X_1$ und $X_2$ ein Chlor-, Brom- oder Jodatom bedeuten.

**4.** Verbindungen der Formel I, wie in Anspruch 3 definiert, worin $X_1$ und $X_2$ ein Bromatom bedeuten.

**5.** Verbindungen der Formel I, wie in einem der Ansprüche 1 bis 4 definiert, worin $R_1$ für ein Brom-, Chlor- oder Fluoratom steht.

**6.** Verbindungen der Formel I, wie in einem der Ansprüche 1 bis 5 definiert, worin Y für einen Rest $SO_2alc_1$ steht, wobei $alc_1$ einen Alkyl- oder Alkenylrest mit bis zu 8 Kohlenstoffatomen bedeutet.

**7.** Verbindungen der Formel I, wie in Anspruch 6 definiert, worin Y einen Rest $SO_2CH_3$ wiedergibt.

**8.** Verbindungen der Formel I, wie in einem der Ansprüche 1 bis 5 definiert, worin Y einen Rest $SO_2Ar$ bedeutet, wobei Ar die vorstehend angegebene Bedeutung besitzt.

**9.** Verbindungen der Formel I, wie in Anspruch 1 definiert, mit den folgenden Bezeichnungen:
Cyano-(3-phenoxy-phenyl)-methyl-1R-[1-α(S),3-α-(R)]-2,2-dimethyl-3-[1-(methylsulfonyloxy)-2,2,2-tribromethyl]-cyclopropancarboxylat,
Cyano-[(3-phenoxy-4-fluor)-phenyl]-methyl-1R-[1-α-(S),3-α-(R)]-2,2-dimethyl-3-[2,2,2-tribrom-1-methylsulfonyloxyethyl]-cyclopropan-carboxylat,
Cyano-(3-phenoxyphenyl)-methyl-1R-[1-α,3-α-(RS*, RS*)]-2,2-dimethyl-3-[2-trifluormethyl-2-brom-2-chlor-1-methylsulfonyloxyethyl]-cyclopropan-carboxylat.

**10.** Verfahren zur Herstellung der Verbindungen der Formel I gemäß Anspruch 1, worin $X_1$, $X_2$ und R die in Anspruch 1 angegebene Bedeutung besitzen, $R_1$ die in Anspruch I angegebene Bedeutung besitzt und

außerdem einen Perfluoralkylrest mit bis zu 8 Kohlenstoffatomen bedeuten kann, Y die in Anspruch 1 angegebene Bedeutung besitzt und außerdem einen Rest

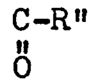

bedeuten kann, worin R" für einen gegebenenfalls substituierten Alkylrest steht,
dadurch **gekennzeichnet,** daß man eine Verbindung der Formel (II)

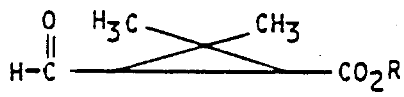

(II)

worin R die vorstehend angegebene Bedeutung besitzt, der Einwirkung einer Verbindung der Formel V

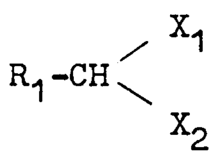

(V)

worin $X_1$, $X_2$ und $R_1$ die vorstehend angegebene Bedeutung besitzen, in Gegenwart einer Base unterzieht, um zu der Verbindung der Formel IV

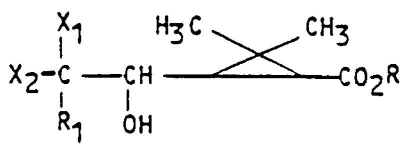

(IV)

zu gelangen, die man der Einwirkung eines Sulfonylierungs- oder Phosphorylierungs- oder Thiophosphorylierungs- oder Acylierungsmittels unterzieht, um zu der entsprechenden Verbindung der Formel I

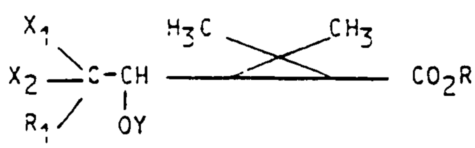

zu gelangen, die man gegebenenfalls in die entsprechende Säure überführt, welche man gegebenenfalls einem Veresterungsmittel unterzieht, um erneut eine Verbindung der Formel I zu erhalten.

**Patentansprüche für folgenden Vertragsstaat : ES**

**1.** Verfahren zur Herstellung der Verbindungen der Formel (I)

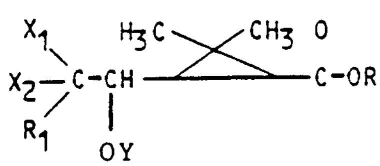

(I)

worin $X_1$ und $X_2$, die identisch oder voneinander verschieden sind, für ein Halogenatom stehen, $R_1$ ein Halogenatom, einen Alkylrest mit bis zu 8 Kohlenstoffatomen, einen gegebenenfalls substituierten Arylrest mit bis zu 14 Kohlenstoffatomen, einen Perfluoralkylrest mit bis zu 8 Kohlenstoffatomen, einen Rest -C≡N, einen Rest

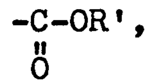

worin R' für einen Alkylrest mit bis zu 8 Kohlenstoffatomen steht, bedeutet, Y für einen Rest steht, ausgewählt unter

- den Resten $SO_2alc_1$, wobei $alc_1$ einen gesättigten oder ungesättigten, linearen oder verzweigten Alkylrest bedeutet, der gegebenenfalls durch eine oder mehrere funktionelle Gruppen substituiert ist;
- den Resten $SO_2A_r$, wobei $A_r$ für einen Arylrest mit bis zu 14 Kohlenstoffatomen steht, der gegebenenfalls durch eine oder mehrere funktionelle Gruppen substituiert ist;
- den Resten

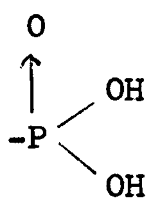 oder 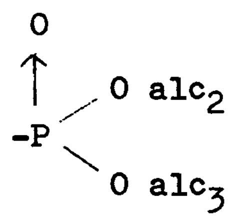 ,

wobei $alc_2$ und $alc_3$ identisch oder voneinander verschieden sind und einen gesättigten oder ungesättigten, linearen oder verzweigten Alkylrest mit bis zu 8 Kohlenstoffatomen wiedergeben, der gegebenenfalls durch eine oder mehrere funktionelle Gruppen substituiert ist, oder $alc_2$ und $alc_3$ mit dem Rest

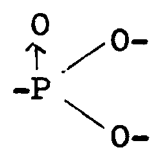

einen Ring

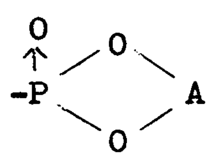

bilden können, wobei A eine gesättigte oder ungesättigte, lineare oder verzweigte Kohlenstoffkette, die gegebenenfalls durch eine oder mehrere funktionelle Gruppen substituiert ist und bis zu 6 Kohlenstoffatome aufweist, wiedergibt;

- den Resten

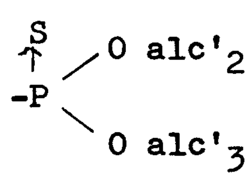 ,

wobei alc'$_2$ und alc'$_3$ die Bedeutungen von alc$_2$ und alc$_3$ besitzen;
- dem Rest

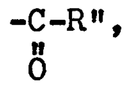

wobei R" einen Arylrest bedeutet; und R bedeutet:
- entweder ein Wasserstoffatom,
- oder einen gegebenenfalls substituierten, linearen oder verzweigten Alkylrest mit 1 bis 8 Kohlenstoffatomen,
- oder einen Benzylrest, der gegebenenfalls substituiert ist durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,
- oder eine Gruppe

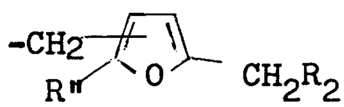

  worin der Substituent R" ein Wasserstoffatom oder eine Methylgruppe bedeutet und der Substituent R$_2$ einen mono-cyclischen Arylrest oder eine Gruppe -C≡CH bedeutet, und insbesondere eine 5-Benzyl-3-furylmethylgruppe
- oder eine Gruppe

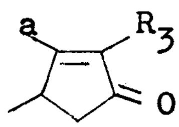

  worin a ein Wasserstoffatom oder eine Methylgruppe bedeutet und R$_3$ einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2-CH=CH-CH_2-CH_3$, $-CH_2-C{\equiv}CH$ wiedergibt,
- oder eine Gruppe

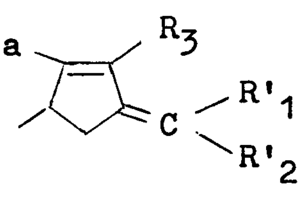

  worin a für ein Wasserstoffatom oder eine Methylgruppe steht, R$_3$ die vorstehend angegebene Bedeutung besitzt, R'$_1$ und R'$_2$ identisch oder voneinander verschieden sind und ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,
- oder eine Gruppe

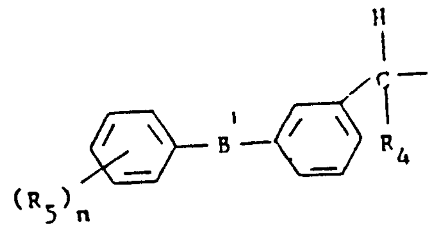

worin B' ein Sauerstoff- oder Schwefelatom, eine Gruppe

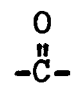

oder $-CH_2$ oder eine Sulfoxidgruppe oder eine Sulfongruppe wiedergibt und $R_4$ ein Wasserstoffatom, einen Rest -C≡N, eine Methylgruppe, einen Rest $-CONH_2$, einen Rest $-CSNH_2$ oder einen Rest -C≡CH bedeutet, $R_5$ für ein Halogenatom oder eine Methylgruppe steht und n eine ganze Zahl entsprechend 0, 1 oder 2 wiedergibt, und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzylgruppe,

- oder eine Gruppe

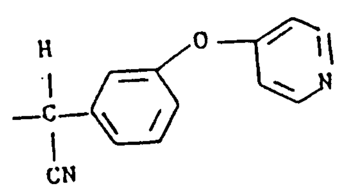

- oder eine Gruppe

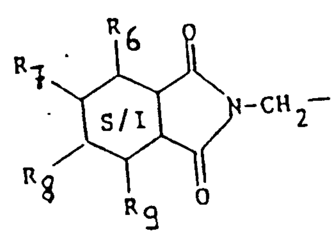

worin die Substituenten $R_6$, $R_7$, $R_8$, $R_9$ für ein Wasserstoffatom, ein Chloratom oder einen Methylrest stehen und worin S/I einen aromatischen Ring oder einen analogen Dihydro- oder Tetrahydroring symbolisiert,

- oder eine Gruppe

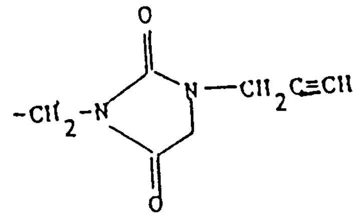

- oder eine Gruppe

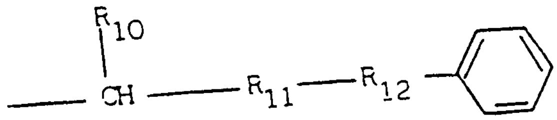

worin $R_{10}$ für ein Wasserstoffatom oder eine CN-Gruppe steht, $R_{12}$ einen Rest $-CH_2$ oder ein Sauerstoffatom bedeutet, $R_{11}$ eine Thiazolyl- oder Thiadiazolylgruppe wiedergibt, deren Bindung mit

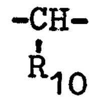

sich in irgendeiner der verfügbaren Positionen befinden kann, wobei $R_{12}$ an $R_{11}$ über ein Kohlenstoffatom zwischen dem Schwefelatom und einem Stickstoffatom gebunden ist,

- oder eine Gruppe

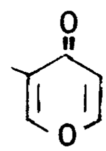

- oder eine Gruppe

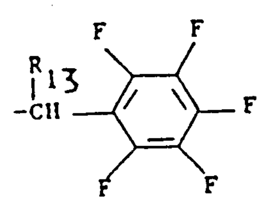

worin $R_{13}$ für ein Wasserstoffatom oder eine CN-Gruppe steht,

- oder eine Gruppe

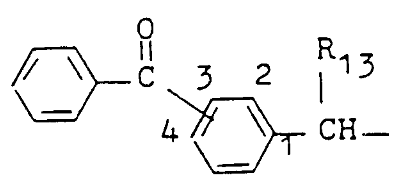

worin $R_{13}$ wie vorstehend definiert ist und der Benzoylrest sich in 3- oder 4-Stellung befindet,

- oder eine Gruppe

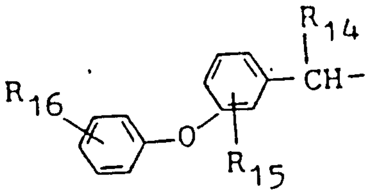

worin $R_{14}$ ein Wasserstoffatom, eine Methyl-, Ethinyl- oder Cyanogruppe bedeutet und $R_{15}$ und $R_{16}$, die voneinander verschieden sind, für ein Wasserstoff-, Fluor- oder Bromatom stehen,

- oder eine Gruppe

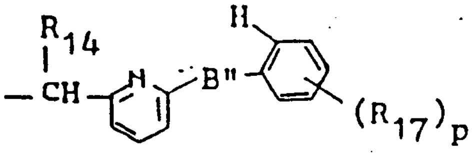

worin $R_{14}$ wie vorstehend definiert ist, jede der Gruppen $R_{17}$ unabhängig einen Alkylrest mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, 3,4-Methylendioxy, Chlor, Fluor oder Brom bedeutet, p für eine ganze Zahl entsprechend 0, 1 oder 2 steht und B" ein Sauerstoff- oder ein Schwefelatom wiedergibt,

- oder eine Gruppe

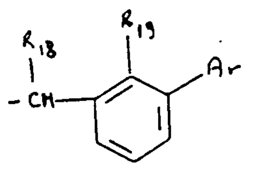

worin $R_{18}$ für ein Wasserstoffatom, eine Methyl-, Ethinyl- oder Cyanogruppe steht und $R_{19}$, welches verschieden ist, ein Wasserstoff-, Fluor- oder Bromatom bedeutet und Ar für einen Arylrest mit bis zu 14 Kohlenstoffatomen steht,

- oder eine Gruppe

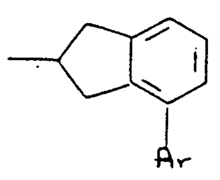

oder

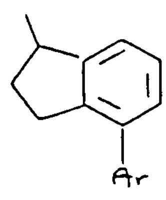

- oder eine Gruppe

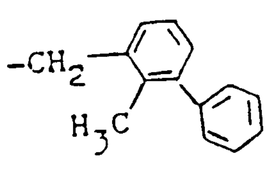

- oder eine Gruppe

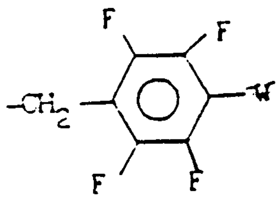

mit W = H, $CH_3$, $OCH_3$

unter der Bedingung, daß $R_1$ keine $CF_3$-Gruppe bedeutet, wenn R = H, Alkyl,
dadurch **gekennzeichnet,** daß man eine Verbindung der Formel (II)

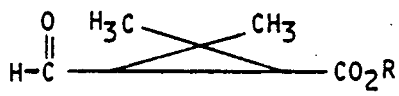

(II)

worin R die vorstehend angegebene Bedeutung besitzt, der Einwirkung einer Verbindung der Formel V

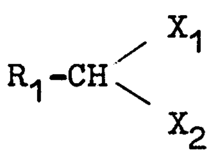

(V)

worin $X_1$, $X_2$ und $R_1$ die vorstehend angegebene Bedeutung besitzen, in Gegenwart einer Base unterzieht, um zu der Verbindung der Formel IV

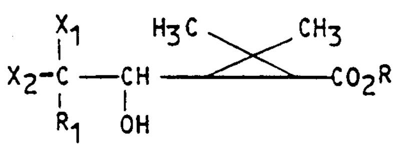

(IV)

zu gelangen, die man der Einwirkung eines Sulfonylierungs- oder Phosphorylierungs- oder Thiophosphorylierungs- oder Acylierungsmittels unterzieht, um zu der entsprechenden Verbindung der Formel I zu gelangen, die man gegebenenfalls in die entsprechende Säure überführt, welche man gegebenenfalls einem Veresterungsmittel unterzieht, um erneut eine Verbindung der Formel I zu erhalten.

**2.** Verfahren gemäß Anspruch 1, dadurch gekennzeichnet, daß man von einer Verbindung der Formel (V) ausgeht, worin $X_1$ und $X_2$ für ein Chlor-, Brom- oder Jodatom stehen.

**3.** Verfahren gemäß Anspruch 2, dadurch gekennzeichnet, daß man von einer Verbindung der Formel (V) ausgeht, worin $X_1$ und $X_2$ für ein Bromatom stehen.

**4.** Verfahren gemäß einem der Ansprüche 1 bis 3, dadurch gekennzeichnet, daß man von einer Verbindung der Formel (V) ausgeht, worin $R_1$ für ein Brom-, Chlor- oder Fluoratom steht.

**5.** Verfahren gemäß einem der Ansprüche 1 bis 3, dadurch gekennzeichnet, daß man von einer Verbindung der Formel (V) ausgeht, worin $R_1$ für eine Trifluormethylgruppe steht.

**6.** Verfahren gemäß einem der Ansprüche 1 bis 5, dadurch gekennzeichnet, daß man von einer Verbindung der Formel (II) ausgeht, worin R bedeutet:
- entweder ein Wasserstoffatom

- oder einen gegebenenfalls substituierten, linearen oder verzweigten Alkylrest mit 1 bis 8 Kohlenstoffatomen,
- oder einen Benzylrest, der gegebenenfalls durch einen oder mehrere Reste substituiert ist, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,
- oder eine Gruppe

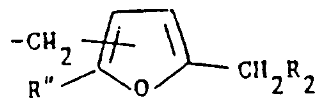

worin der Substituent R" für ein Wasserstoffatom oder eine Methylgruppe steht und der Substituent $R_2$ einen monocyclischen Arylrest oder eine Gruppe $-CH_2{\equiv}CH$ bedeutet, und insbesondere eine 5-Benzyl-3-furylmethylgruppe
- oder eine Gruppe

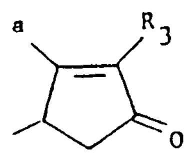

worin a für ein Wasserstoffatom oder einen Methylrest steht und $R_3$ einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2-CH=CH-CH_2-CH_3$, $-CH_2-C{\equiv}CH$ bedeutet,
- oder eine Gruppe

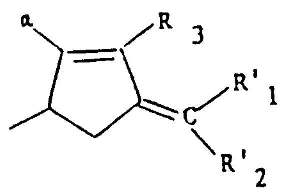

worin a ein Wasserstoffatom oder eine Methylgruppe wiedergibt, $R_3$ die vorstehend angegebene Bedeutung besitzt, $R'_1$ und $R'_2$, die identisch oder verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,
- oder eine Gruppe

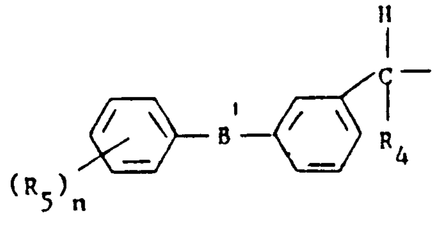

worin B' für ein Sauerstoff- oder Schwefelatom, eine Gruppe

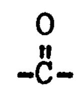

oder $-CH_2$ oder eine Sulfoxidgruppe oder eine Sulfongruppe steht und $R_4$ ein Wasserstoffatom, einen Rest $-C{\equiv}N$, eine Methylgruppe, einen Rest $-CONH_2$, einen Rest $-CSNH_2$ oder einen Rest $-C{\equiv}CH$ wiedergibt, $R_5$ ein Halogenatom oder einen Methylrest bedeutet und n eine Zahl entsprechend 0, 1 oder 2 wiedergibt und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxyphenyl)-ethyl- oder α-Thioamido-3-phenoxybenzylgruppe,

- oder eine Gruppe

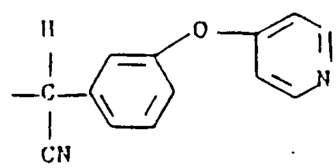

- oder eine Gruppe

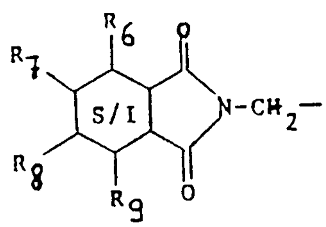

worin die Substituenten $R_6$, $R_7$, $R_8$, $R_9$ ein Wasserstoffatom, ein Chloratom oder einen Methylrest wiedergeben und worin S/I einen aromatischen Ring oder einen analogen Dihydro- oder Tetrahydroring symbolisiert,

- oder eine Gruppe

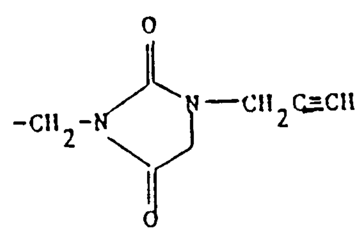

- oder eine Gruppe

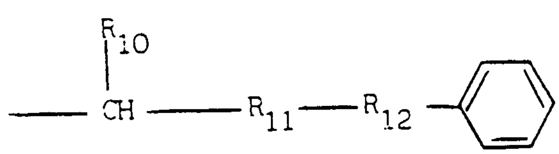

worin $R_{10}$ ein Wasserstoffatom oder eine CN-Gruppe bedeutet, $R_{12}$ einen Rest $-CH_2-$ oder ein Sauerstoffatom wiedergibt, $R_{11}$ für eine Thiazolyl- oder Thiadiazolylgruppe steht, deren Bindung mit

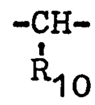

sich in einer der verfügbaren Positionen befinden kann, wobei $R_{12}$ an $R_{11}$ über ein Kohlenstoffatom zwischen dem Schwefelatom und einem Stickstoffatom gebunden ist,

- oder eine Gruppe

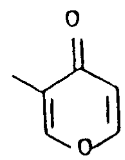

- oder eine Gruppe

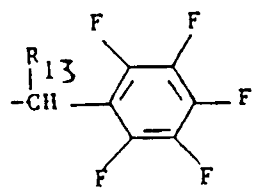

worin $R_{13}$ für ein Wasserstoffatom oder eine CN-Gruppe steht,

- oder eine Gruppe

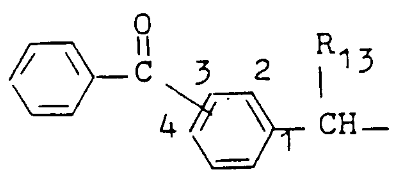

worin $R_{13}$ wie vorstehend definiert ist und der Benzoylrest sich in 3- oder 4-Stellung befindet,

- oder eine Gruppe

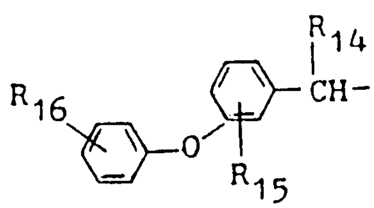

worin $R_{14}$ für ein Wasserstoffatom, eine Methyl-, Ethinyl- oder Cyanogruppe steht und $R_{15}$ und $R_{16}$ voneinander verschieden sind und ein Wasserstoff-, Fluor- oder Bromatom bedeuten,

- oder eine Gruppe

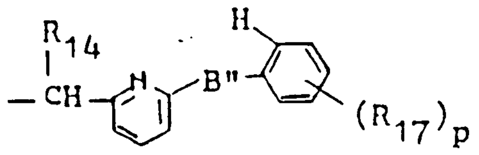

worin $R_{14}$ wie vorstehend definiert ist, eine jede der Gruppen $R_{17}$ unabhängig eine Alkylgruppe mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, 3,4-Methylendioxy, Chlor, Fluor oder Brom bedeutet, p eine ganze Zahl entsprechend 0, 1 oder 2 wiedergibt und B" für ein Sauerstoff- oder ein Schwefelatom steht,

- oder eine Gruppe

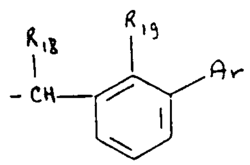

worin $R_{18}$ ein Wasserstoffatom, eine Methyl-, Ethinyl- oder Cyanogruppe bedeutet und $R_{19}$, welches verschieden ist, ein Wasserstoff-, Fluor- oder Bromatom bedeutet und Ar für eine Arylgruppe mit bis zu 14 Kohlenstoffatomen steht,

- oder eine Gruppe

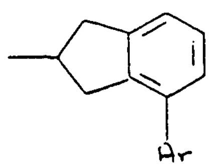

oder

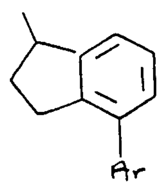

.

**7.** Verfahren gemäß Anspruch 6, dadurch gekennzeichnet, daß man von einer Verbindung der Formel (II) ausgeht, worin R einen der folgenden Reste bedeutet:

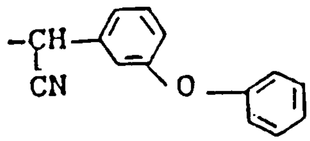

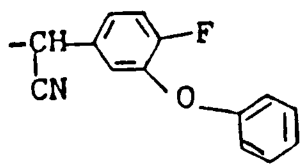

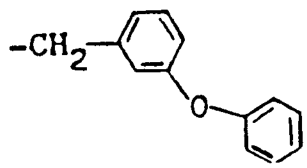

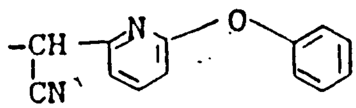

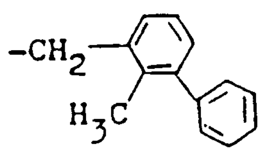

oder 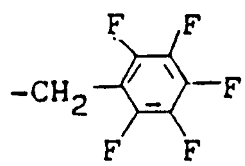 oder 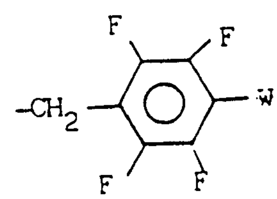

mit W=H, $CH_3$, $OCH_3$

oder 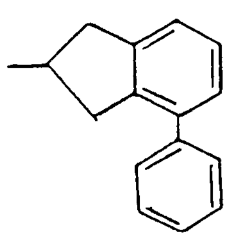

**8.** Verfahren gemäß Anspruch 1, dadurch gekennzeichnet, daß man die Verbindung der Formel (IV) der Einwirkung eines Sulfonylierungsmittels unterzieht, das derart ausgewählt ist, daß man die Verbindungen der Formel (I) herstellt, worin Y einen Rest $SO_2alc_1$ bedeutet, wobei $alc_1$ für einen Alkyl- oder Alkenylrest mit bis zu 8 Kohlenstoffatomen steht.

**9.** Verfahren gemaß Anspruch 8, dadurch gekennzeichnet, daß man die Verbindung der Formel (IV) der Einwirkung eines Sulfonylierungsmittels unterzieht, das derart ausgewählt ist, daß man die Verbindungen der Formel (I) herstellt, worin Y für einen Rest $SO_2CH_3$ steht.

**10.** Verfahren gemäß Anspruch 1, dadurch gekennzeichnet, daß man die Verbindung der Formel (IV) der Einwirkung eines Sulfonylierungsmittels unterzieht, das derart ausgewählt ist, daß man die Verbindungen der Formel (I) herstellt, worin Y fur einen Rest $SO_2Ar$ steht, wobei Ar die vorstehend angegebene Bedeutung besitzt.

**11.** Verwendung der Verbindungen der Formel I, wie in einem der Ansprüche 1 bis 10 definiert, dadurch gekennzeichnet, daß man eine Verbindung der Formel I der Einwirkung eines Reduktionsmittels unterzieht, um die entsprechende Verbindung der Formel III

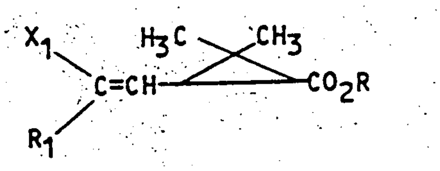

(III)

zu erhalten, worin $X_1$, $R_1$ und R die vorstehend angegebene Bedeutung besitzen.

**12.** Verwendung gemaß Anspruch 11, dadurch gekennzeichnet, daß die Verbindung der Formel I der Formel I' entspricht

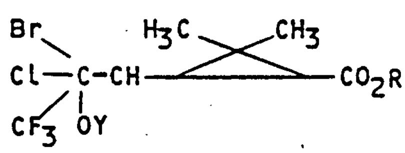

(I')

worin Y und R die vorstehend angegebene Bedeutung besitzen,und die Verbindung der Formel III derjenigen der Formel III'

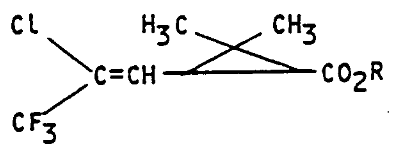

(III')

entspricht.

**13.** Verwendung gemäß Anspruch 11, dadurch gekennzeichnet, daß die Verbindung der Formel I der Formel I"

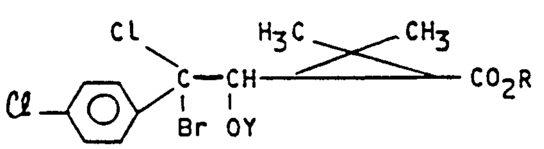

(I")

entspricht, worin Y und R die vorstehend angegebene Bedeutung besitzen, und die erhaltene Verbindung III derjenigen der Formel III"

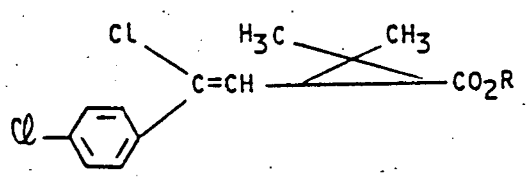

(III")

entspricht.